(19) Europäisches Patentamt
European Patent Office
Office européen des brevets

(11) Veröffentlichungsnummer: **0 046 961**
**B1**

(12) # EUROPÄISCHE PATENTSCHRIFT

(45) Veröffentlichungstag der Patentschrift: **13.02.85**

(21) Anmeldenummer: **81106557.2**

(22) Anmeldetag: **24.08.81**

(51) Int. Cl.[4]: **C 07 D 303/48,** A 61 K 31/335 // C07C69/608, C07C69/708, C07C69/734, C07C19/02, C07C43/12, C07C43/13

(54) **Epoxi-cycloalkylalkancarbonsäuren, Verfahren zu ihrer Herstellung, ihre Verwendung sowie sie enthaltende Arzneimittel.**

(30) Priorität: **29.08.80 DE 3032668**
**25.02.81 CH 1243/81**

(43) Veröffentlichungstag der Anmeldung: **10.03.82 Patentblatt 82/10**

(45) Bekanntmachung des Hinweises auf die Patenterteilung: **13.02.85 Patentblatt 85/7**

(84) Benannte Vertragsstaaten: **AT BE CH DE FR GB IT LI LU NL SE**

(56) Entgegenhaltungen:
**EP - A - 0 025 192**
**GB - A - 1 551 078**

**MEDICINAL CHEMISTRY 3rd Edition, pp. 74-76**

**Die Akte enthält technische Angaben, die nach dem Eingang der Anmeldung eingereicht wurden und die nicht in dieser Patentschrift enthalten sind.**

(73) Patentinhaber: **Byk Gulden Lomberg Chemische Fabrik GmbH, Byk-Gulden-Strasse 2, D-7750 Konstanz (DE)**

(72) Erfinder: **Eistetter, Klaus, Dr., Säntisblick 7, D-7750 Konstanz 19 (DE)**
Erfinder: **Ludwig, Gerhard, Dr., Meisenweg 6, D-7750 Konstanz 16 (DE)**
Erfinder: **Rapp, Erich, Dr., Pfarrer-Braun-Strasse 6, D-7760 Radolfzell 17 (DE)**
Erfinder: **Wolf, Horst, Dr., Brandesstrasse 29, D-7750 Konstanz (DE)**

**EP 0 046 961 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Epoxi-cycloalkylalkancarbonsäuren, Verfahren zu ihrer Herstellung, ihre Verwendung sowie sie enthaltende Arzneimittel.

Die erfindungsgemäßen Verbindungen werden in der pharmazeutischen Industrie als Zwischenprodukte und zur Herstellung von Medikamenten verwendet.

Aus der GB-A-1 551 078 sind Alkyl-oxirancarbonsäureester mit hypoglycämischer Wirksamkeit bekannt. Aufgabe der vorliegenden Erfindung war es, neue Verbindungen mit hypoglycämischer und hypoketonämischer Wirkung zu schaffen.

Gegenstand der Erfindung sind Epoxi-cycloalkylalkancarbonsäuren der allgemeinen Formel I

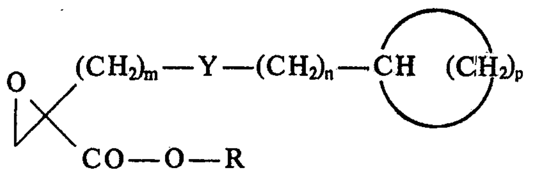

(I)

worin

R ein Wasserstoffatom oder eine Niederalkylgruppe ($C_1-C_4$),
Y ein Sauerstoffatom (—O—) oder eine Methylengruppe ($-CH_2-$),
m eine ganze Zahl von 0 bis 6,
n eine ganze Zahl von 0 bis 6 und
p eine ganze Zahl von 2 bis 11

bedeutet (wobei m nicht 0 oder 1 sein kann, wenn Y ein Sauerstoffatom darstellt und wobei die Summe der Zahlen m, n udn p eine ganze Zahl von 6 bis 15 ist), sowie die Salze der Carbonsäuren.

Als Niederalkylgruppe kommen geradkettige oder verzweigte Alkylreste mit 1 bis 4 Kohlenstoffatomen in Frage: Geradkettige Alkylreste sind der Methyl-, Ethyl-, n-Propyl- und n-Butylrest, von denen der Methyl- und der Ethylrest bevorzugt sind. Verzweigte Alkylreste sind beispielsweise der Isopropyl-, Isobutyl- und sec.-Butylrest, von denen der Isopropylrest bevorzugt ist.

Als Salze kommen Salze mit anorganischen und organischen Basen in Betracht. Pharmakologisch nicht verträgliche Salze werden nach an sich bekannten Methoden in pharmakologisch, d. h. biologisch, verträgliche Salze übergeführt, die unter den erfindungsgemäßen Salzen bevorzugt sind.

Als Kationen für die Salzbildung werden vor allem die Kationen der Alkalimetalle, Erdalkalimetalle oder Erdmetalle verwendet, es kommen jedoch auch die entsprechenden Kationen organischer Stickstoffbasen, wie Amine, Aminoalkanole, Aminozucker, basische Aminosäuren, etc. zur Anwendung.

Beispielsweise seien die Salze mit Lithium, Natrium, Kalium, Magnesium, Calcium, Aluminium, Ethylendiamin, Dimethylamin, Diethylamin, Morpholin, Piperidin, Piperazin, N-Niedrigalkylpiperazin (z. B. N-Methylpiperazin), Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Glucamin, N-Methylglucamin, Glucosamin, N-Methylglucosamin, Lysin, Ornithin, Arginin und Chinolin genannt.

Eine Ausgestaltung der Erfindung sind Epoxi-cycloalkylalkancarbonsäuren der allgemeinen Formel I*

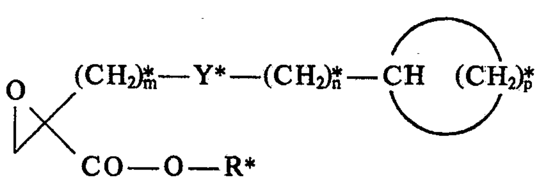

(I*)

worin

R* ein Wasserstoffatom oder eine Niederalkylgruppe ($C_1-C_4$),
Y* eine Methylengruppe ($-CH_2-$),
m* eine ganze Zahl von 1 bis 6,
n* die Zahl 1 und
p* eine ganze Zahl von 4 bis 7

bedeutet (wobei die Summe der Zahlen m*, n* und p* eine ganze Zahl von 6 bis 11 ist), sowie die Salze der Carbonsäuren.

Bevorzugte Vertreter dieser Ausgestaltung sind solche der Formel I*, worin R* ein Wasserstoffatom, eine Methyl- oder Ethylgruppe, Y* eine Methylengruppe ($-CH_2-$), m* eine ganze Zahl von 2 bis 4, n* die Zahl 1 und p* die Zahl 5 bedeutet, sowie die Salze der Carbonsäuren.

Eine weitere Ausgestaltung der Erfindung sind Epoxi-cycloalkylalkancarbonsäuren der allgemeinen Formel I**

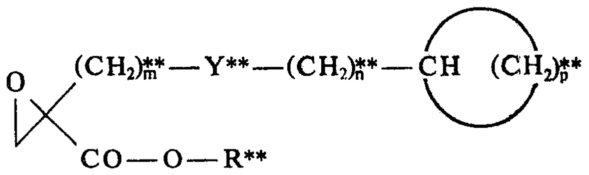

(I**)

worin

R** ein Wasserstoffatom oder eine Niederalkylgruppe ($C_1-C_4$),
Y** ein Sauerstoffatom (—O—),
m** eine ganze Zahl von 2 bis 6,
n** eine ganze Zahl von 0 bis 4 und
p** eine ganze Zahl von 4 bis 7

bedeutet (wobei die Summe der Zahlen m**, n** und p** eine ganze Zahl von 6 bis 11 ist), sowie die Salze der Carbonsäuren.

Bevorzugte Vertreter dieser Ausgestaltung sind solche der Formel I**, worin R** ein Wasserstoffatom, eine Methyl- oder Ethylgruppe, Y** ein Sauerstoffatom (—O—), m** eine ganze Zahl von 2 bis 5, n** eine ganze Zahl von 0 bis 3 und p** die Zahl 5 bedeutet, (wobei die Summe aus m** und n** eine Zahl von 3 bis 5 ist), sowie die Salze der Carbonsäuren.

Als Vertreter der erfindungsgemäßen Verbindungen seien beispielsweise

2-(4-Cyclohexylbutyl)-oxiran-2-carbonsäureethylester,
2-(5-Cyclopropylpentyl)-oxiran-2-carbonsäuremethylester,
2-(3-Cyclooctylpropyl)-oxiran-2-carbonsäureethylester,
2-(8-Cyclopentyloctyl)-oxiran-2-carbonsäuremethylester,
2-(6-Cyclohexylhexyl)-oxiran-2-carbonsäure-n-butylester,
2-(10-Cyclopentyldecyl)-oxiran-2-carbonsäureethylester,
2-(2-Cycloheptylethyl)-oxiran-2-carbonsäureisopropylester,
2-(7-Cyclobutylheptyl)-oxiran-2-carbonsäuremethylester,
2-Cyclononylmethyl-oxiran-2-carbonsäure-sec.-butylester,
2-(9-Cyclopropylnonyl)-oxiran-2-carbonsäuremethylester,
2-(5-Cyclohexylpentyl)-oxiran-2-carbonsäure-n-propylester,
2-(4-Cyclooctylbutyl)-oxiran-2-carbonsäuremethylester,
2-(4-Cyclopentylbutyl)-oxiran-2-carbonsäureisopropylester,
2-(6-Cycloheptylhexyl)-oxiran-2-carbonsäuremethylester,
2-(5-Cyclobutylpentyl)-oxiran-2-carbonsäureethylester,
2-(5-Cyclooctylpentyl-oxiran-2-carbonsäure-n-propylester,
2-(7-Cycloheptylheptyl)-oxiran-2-carbonsäuremethylester,
2-(5-Cyclododecylpentyl)-oxiran-2-carbonsäureethylester,
2-(6-Cyclooctylhexyl)-oxiran-2-carbonsäuremethylester,
2-(5-Cycloheptylpentyl)-oxiran-2-carbonsäure-sec.-butylester,
2-(2-Cyclohexylethyl)-oxiran-2-carbonsäureethylester,
2-(6-Cyclopentylhexyl)-oxiran-2-carbonsäuremethylester,
2-(7-Cyclohexylheptyl)-oxiran-2-carbonsäureisopropylester,
2-(4-Cyclobutylbutyl)-oxiran-2-carbonsäure-n-butylester,
2-(2-Cyclononylethyl)-oxiran-2-carbonsäureethylester,
2-(4-Cyclododecylbutyl)-oxiran-2-carbonsäuremethylester,
2-(6-Cyclobutylhexyl)-oxiran-2-carbonsäureisopropylester,
2-(7-Cyclooctylheptyl)-oxiran-2-carbonsäuremethylester,
2-(2-Cyclopentylethyl)-oxiran-2-carbonsäureethylester,
2-(8-Cyclohexyloctyl)-oxiran-2-carbonsäure-n-propylester,
2-(4-Cycloheptylbutyl)-oxiran-2-carbonsäuremethylester,
2-(7-Cyclopentylheptyl)-oxiran-2-carbonsäuremethylester,
2-(6-Cyclobutylhexyl)-oxiran-2-carbonsäureethylester,
2-(2-Cyclooctylethyl)-oxiran-2-carbonsäure-n-propylester,
2-[3-(2-Cyclohexylethoxy)-propyl]-oxiran-2-carbonsäureethylester,
2-[2-(3-Cyclobutylpropyloxy)-ethyl]-oxiran-2-carbonsäuremethylester,2-[4-(2-Cycloheptylethoxy)-butyl]-oxiran-2-carbonsäureisopropylether,
2-[3-(3-Cyclopentylpropyloxy)-propyl]-oxiran-2-carbonsäure-n-butylester,
2-[3-(Cyclohexyloxy)-propyl]-oxiran-2-carbonsäureethylester,
2-[6-(4-Cyclopropylbutyloxy)-hexyl]-oxiran-2-carbonsäuremethylester,2-[5-(Cyclopenty-

loxy)-pentyl]-oxiran-2-carbonsäureethylester,
2-[2-(Cyclooctylmethoxy)-ethyl]-oxiran-2-carbonsäureisopropylester,
2-[3-(2-Cyclononylethoxy)-propyl]-oxiran-2-carbonsäuremethylester,
2-[2-(Cyclododecylmethoxy)-ethyl]-oxiran-2-carbonsäureethylester,
2-[6-(3-Cyclopropylpropyloxy)-hexyl]-oxiran-2-carbonsäure-n-butylester,
2-[5-(4-Cyclobutylbutyloxy)-pentyl]-oxiran-2-carbonsäureethylester,
2-[3-(2-Cyclododecylethoxy)-propyl]-oxiran-2-carbonsäuremethylester,2-[2-(6-Cyclopentylhexyloxy)-ethyl]-oxiran-2-carbonsäure-n-propylester,
2-[6-(Cyclohexylmethoxy)-hexyl]-oxiran-2-carbonsäureisopropylester,
2-[5-(Cycloheptyloxy)-pentyl]-oxiran-2-carbonsäuremethylester,
2-[3-(Cycloundecyloxy)-propyl]-oxiran-2-carbonsäureethylester,
2-[4-(Cyclooctylmethoxy)-butyl]-oxiran-2-carbonsäure-n-butylester,
2-[2-(5-Cyclobutylpentyloxy)-ethyl]-oxiran-2-carbonsäureethylester,
2-[2-(Cyclononylmethoxy)-ethyl]-oxiran-2-carbonsäure-n-propylester,
2-[5-(2-Cycloheptylethoxy)-pentyl]-oxiran-2-carbonsäuremethylester,
2-[6-(2-Cyclopropylethoxy)-hexyl]-oxiran-2-carbonsäureisopropylester,
2-[3-(6-Cyclopentylhexyloxy)-propyl]-oxiran-2-carbonsäuremethylester,
2-[4-(3-Cyclohexylpropyloxy)-butyl]-oxiran-2-carbonsäure-n-propylester,
2-[5-(Cycloheptylmethoxy)-pentyl]-oxiran-2-carbonsäuremethylester,
2-[6-(Cyclooctyloxy)-hexyl]-oxiran-2-carbonsäureethylester,
2-[4-(Cyclononyloxy)-butyl]-oxiran-2-carbonsäure-sec.-butylester,
2-[2-(4-Cyclohexylbutyloxy)-ethyl]-oxiran-2-carbonsäuremethylester,
2-[4-(2-Cyclopentylethoxy)-butyl]-oxiran-2-carbonsäureethylester,
2-[4-(Cyclopentylmethoxy)-butyl]-oxiran-2-carbonsäureisopropylester,2-[5-(Cyclohexyloxy)-pentyl]-oxiran-2-carbonsäureethylester,
2-[3-(3-Cycloheptylpropyloxy)-propyl]-oxiran-2-carbonsäuremethylester,
2-[3-(Cyclododecyloxy)-propyl]-oxiran-2-carbonsäure-n-butylester,
2-[4-(2-Cyclooctylethoxy)-butyl]-oxiran-2-carbonsäureethylester,
2-[2-(4-Cycloheptylbutyloxy)-ethyl]-oxiran-2-carbonsäuremethylester,2-[2-(4-Cyclopentylbutyloxy)-ethyl]-oxiran-2-carbonsäureisopropylester,
2-[2-(5-Cyclohexylpentyloxy)-ethyl]-oxiran-2-carbonsäureethylester,
2-[6-(Cycloheptyloxy)-hexyl]-oxiran-2-carbonsäureisopropylester,
2-[2-(3-Cyclooctylpropyloxy)-ethyl]-oxiran-2-carbonsäure-n-butylester,
2-[2-(2-Cyclohexylethoxy)-ethyl]-oxiran-2-carbonsäuremethylester,
2-[2-(2-Cycloheptylethoxy)-ethyl]-oxiran-2-carbonsäureisopropylester,
2-[4-(Cycloheptyloxy)-butyl]-oxiran-2-carbonsäureethylester,
2-[2-(Cyclooctyloxy)-ethyl]-oxiran-2-carbonsäuremethylester,
2-[3-(Cyclopentyloxy)-propyl]-oxiran-2-carbonsäure-n-butylester,
2-[6-(Cyclopentylmethoxy)-hexyl]-oxiran-2-carbonsäureethylester,

die entsprechenden Oxiran-2-carbonsäuren sowie deren Salze mit anorganischen und organischen Basen genannt.

Die Epoxi-cycloalkylalkancarbonsäuren der allgemeinen Formel I bzw. der Ausgestaltungen I* und I** besitzen ein Chiralitätszentrum. Die Erfindung schließt daher sowohl die Racemate und die Enantiomeren als auch deren Gemische ein.

Die erfindungsgemäßen Verbindungen weisen wertvolle pharmakologische Eigenschaften auf, die sie gewerblich verwertbar machen. Sie wirken hypoglycämisch und hypoketonämisch mit vergleichsweise kurzzeitiger Wirkungsdauer.

Aufgrund ihrer vorteilhaften Wirksamkeit sind die erfindungsgemäßen Epoxi-cycloalkylalkancarbonsäuren der allgemeinen Formel I bzw. der Ausgestaltungen I* und I** sowie die pharmakologisch verträglichen Salze zur human- und veterinärmedizinischen Behandlung und Prophylaxe von Krankheiten, die auf Störungen des Glucose- und Fettstoffwechsels beruhen, geeignet. Beispielsweise werden behandelt prädiabetische Zustände zur Verhinderung der Manifestation des Diabetes, manifester Diabetes, z. B. Erwachsenendiabetes, labiler Diabetes von Jugendlichen sowie alle krankhaften Zustände, die mit einer pathologisch erhöhten Ketonkörperproduktion einhergehen.

Die Applikation der erfindungsgemäßen Verbindungen stellt somit ein Verfahren zur Bekämpfung der angegebenen Krankheiten dar. Gegenstand der Erfindung sind daher auch die erfindungsgemäßen Verbindungen zur Anwendung bei der Bekämpfung der angegebenen Krankheiten.

Ein weiterer Gegenstand der Erfindung sind Arzneimittel, die eine oder mehrere der Epoxi cycloal kylalkancarbonsäuren der allgemeinen Formel I

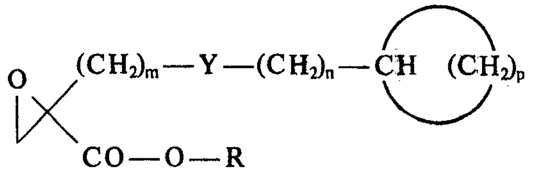

(I)

worin

R ein Wasserstoffatom oder eine Niederalkylgruppe ($C_1-C_4$),
Y ein Sauerstoffatom ($-O-$) oder eine Methylengruppe ($-CH_2-$),
m eine ganze Zahl von 0 bis 6,
n eine ganze Zahl von 0 bis 6 und
p eine ganze Zahl von 2 bis 11

bedeutet (wobei m nicht 0 oder 1 sein kann, wenn Y ein Sauerstoffatom darstellt und wobei die Summe der Zahlen m, n und p eine ganze Zahl von 6 bis 15 ist), und/oder die pharmakologisch verträglichen Salze der Säuren mit anorganischen oder organischen Basen enthalten.

Ausgestaltungen der Arzneimittel sind solche, die Epoxi-cycloalkylalkancarbonsäuren der Ausgestaltungen I* und I** und/oder die pharmakologisch verträglichen Salze der Säuren mit anorganischen oder organischen Basen enthalten.

Gegenstand der Erfindung ist außerdem die Verwendung der erfindungsgemäßen Verbindungen zur Herstellung von Arzneimitteln zur Bekämpfung der angegebenen Krankheiten.

Die Arzneimittel werden nach an sich bekannten Verfahren hergestellt. Als Arzneimittel können die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 1 bis 95, vorzugsweise 15 bis 85 Gewichtsprozent der Gesamtmischung.

In Übereinstimmung mit der Erfindung können im humanmedizinischen Bereich die Wirkstoffe in beliebiger Form, z. B. systemisch, angewandt werden unter der Voraussetzung, daß die Ausbildung bzw. Aufrechterhaltung von ausreichenden Blut- oder Gewebsspiegeln an Wirkstoffen gewährleistet ist. Das kann beispielsweise durch orale oder parenterale Gabe in geeigneten Dosen erreicht werden. Vorteilhafterweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulats, einer Lösung, einer Emulsion oder einer Suspension sein.

Unter »Einheitsdosis« im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachem einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z. B. in Form einer Tablette mit Bruchkerbe.

Die pharmazeutischen Zubereitungen gemäß der Erfindung enthalten, wenn sie in Einheitsdosen vorliegen und für die Applikation, z. B. am Menschen bestimmt sind, etwa 2 bis 200 mg, vorteilhafterweise 10 bis 100 mg und insbesondere 20 bis 60 mg Wirkstoff.

Im allgemeinen erweist es sich in der Humanmedizin als vorteilhaft, den oder die Wirkstoffe bei oraler Gabe in einer Tagesdosis von etwa 0,1 bis etwa 30, vorzugsweise 0,3 bis 15, insbesondere 0,6 bis 3 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von etwa 0,05 bis etwa 10, vorzugsweise 0,1 bis 5, insbesondere 0,3 bis 1 mg/kg Körpergewicht.

Bei einer parenteralen Behandlung, z. B. einer intravenösen oder intramusculären Applikation können ähnliche Dosierungen zur Anwendung kommen. Bei dieser Therapie werden etwa 0,3 bis 1 mg Wirkstoff/kg Körpergewicht appliziert. Im Bedarfsfall kann die Dosis auf 0,3 bis 15, in besonders akuten Fällen auf 0,3 bis 30 mg Wirkstoff/kg Körpergewicht erhöht werden.

Die therapeutische Verabreichung der pharmazeutischen Zubereitung erfolgt bei Dauermedikation im allgemeinen zu festgelegten Zeitpunkten, wie 1- bis 4mal am Tage, z. B. jeweils nach den Mahlzeiten und/oder am Abend. Bei akuten Anlässen erfolgt die Medikation zu variierendem Zeitpunkt. Unter bestimmten Gegebenheiten kann es erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation

des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikation der Wirkstoffe nimmt der Fachmann aufgrund seines Fachwissens vor.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses für den therapeutisch aktiven Bestandteil in Anwendung kommen. Ein Trägerstoff kann z. B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z. B. Tabletten, Dragées, harte und weiche Kapseln, z. B. aus Gelatine, dispergierbare Pulver, Granulate, wäßrige und ölige Suspensionen, Emulsionen oder Lösungen kommen.

Tabletten können inerte Verdünnungsmittel, z. B. Calciumcarbonat, Calciumphosphat, Natriumphosphat oder Xylit; Granulierungs- und Verteilungsmittel, z. B. Calciumphosphat oder Alginate; Bindemittel, z. B. Stärke, Gelatine oder Akaziengummi; und Gleitmittel, z. B. Aluminium- oder Magnesiumstearat, Talkum oder Silikonöl, enthalten. Sie können zusätzlich mit einem Überzug versehen sein, der auch so beschaffen sein kann, daß eine verzögerte Auflösung und Resorption des Arzneimittels im Gastrointestinaltrakt und damit z. B. eine bessere Verträglichkeit, Protrahierung oder eine Retardierung erreicht wird. Gelatinekapseln können den Arzneistoff vermischt mit einem festen Verdünnungsmittel, z. B. Calciumcarbonat oder Kaolin, oder einem öligen Verdünnungsmittel, z. B. Paraffinöl, enthalten.

Wäßrige Suspensionen können Suspendiermittel, z. B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z. B. Polyoxyethylenstearat, Heptadecaethylenoxycetanol, Polyoxyethylensorbitolmonooleat, Polyoxyethylensorbitanmonooleat oder Lecithin; Konservierungsmittel, z. B. Methyl- oder Propylhydroxybenzoate; Geschmacksmittel; Süßungsmittel, z. B. Saccharin, Natriumcyclamat, enthalten.

Ölige Suspensionen können z. B. Paraffinöl und Verdickungsmittel, wie Bienenwachs, Hartparaffin oder Cetylalkohol, enthalten; ferner Süßungsmittel, Geschmacksmittel und Antioxidantien.

In Wasser dispergierbare Pulver und Granulate können die Arzneistoffe in Mischung mit Dispergier-, Benetzungs- und Suspendiermitteln, z. B. den obengenannten, sowie mit Süßungsmitteln, Geschmacksmitteln und Farbstoffen enthalten.

Emulsionen können z. B. Paraffinöl neben Mulgiermitteln, wie Akaziengummi, Traganthgummi, Phosphatiden, Sorbitanmonooleat, Polyoxyethylensorbitanmonooleat, und Süßungs- und Geschmacksmitteln enthalten.

Zur parenteralen Anwendung der Arzneistoffe dienen steril injizierbare wäßrige Suspensionen, isotonische Salzlösungen oder sonstige Lösungen, die Dispergier- oder Benetzungsmittel und/oder pharmakologisch verträgliche Verdünnungsmittel, z. B. Propylen- oder Butylenglycol, enthalten können.

Der oder die Wirkstoffe können gegebenenfalls mit einem oder mehreren der angegebenen Träger- oder Zusatzstoffe auch in mikroverkapselter Form formuliert werden.

Neben den erfindungsgemäßen Epoxi-cycloalkylalkancarbonsäuren, in denen die Substituenten die oben angegebene Bedeutung haben, und/oder ihren Salzen können die pharmazeutischen Zubereitungen weiterhin einen oder mehrere pharmakologisch aktive Bestandteile anderer Arzneimittelgruppen, wie Antidiabetika (Sulfonamide, Sulfonylharnstoffe u. a.), z. B. Carbutamid, Tolbutamid, Chlorpropamid, Glibenclamid, Glibornurid, Glisoxepid, Gliquidon, Glymidin oder Hypolipidämika, wie Nikotinsäure sowie deren Derivate und Salze, enthalten.

Ein weiterer Gegenstand der Erfindung ist ein Verfahren zur Herstellung von Epoxi-cycloalkylalkancarbonsäuren der allgemeinen Formel I

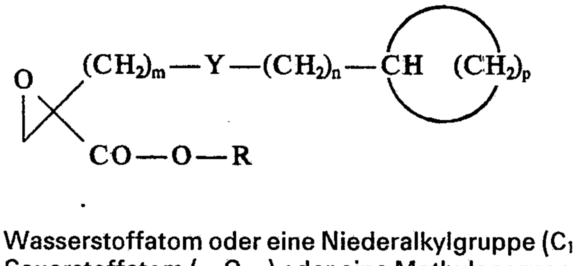

(I)

worin

R ein Wasserstoffatom oder eine Niederalkylgruppe ($C_1-C_4$),
Y ein Sauerstoffatom ($-O-$) oder eine Methylengruppe ($-CH_2-$),
m eine ganze Zahl von 0 bis 6,
n eine ganze Zahl von 0 bis 6 und
p eine ganze Zahl von 2 bis 11

bedeutet (wobei m nicht 0 oder 1 sein kann, wenn Y ein Sauerstoffatom ist und wobei die Summe der Zahlen m, n und p eine ganze Zahl von 6 bis 15 ist), sowie den Salzen der Carbonsäuren, das dadurch gekennzeichnet ist, daß man eine substituierte $\alpha$-Methylencarbonsäure der allgemeinen Formel:

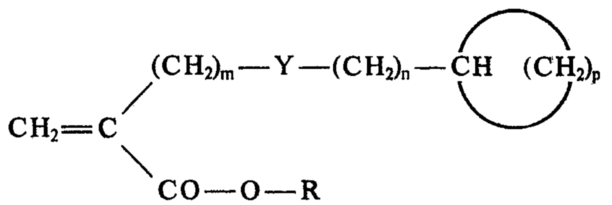

(II)

worin R, Y, m, n und p die vorstehend angegebenen Bedeutungen haben, oxidiert und gegebenenfalls anschließend die erhaltenen Niederalkylester verseift und gewünschtenfalls anschließend in die Salze überführt oder die erhaltenen Säuren in die Salze oder Niederalkylester überführt.

Die Oxidation der $\alpha$-Methylencarbonsäuren II erfolgt unter den Bedingungen, wie sie dem Fachmann für die Oxidation von Kohlenstoff-Kohlenstoff-Doppelbindungen zu Epoxiden bekannt sind. Als Oxidationsmittel kommen beispielsweise Peroxoverbindungen, wie Wasserstoffperoxid, Peressigsäure, Trifluorperessigsäure, 3,5-Dinitroperbenzoesäure, bevorzugt m-Chlorperbenzoesäure oder Permaleinsäure in Betracht. Die Reaktion wird zweckmäßigerweise in inerten Lösungsmitteln, z. B. aromatischen oder chlorierten Kohlenwasserstoffen, wie Benzol, Toluol, Dichlormethan, Chloroform durchgeführt. Die Reaktionstemperaturen liegen zwischen 0° und der Siedetemperatur des Lösungsmittels, bevorzugt zwischen 20° und 70° C.

Die Verseifung der Niederalkylester erfolgt in an sich bekannter Weise. Sie wird beispielsweise mit einer wässerigen oder alkoholischen (z. B. ethanolischen) Alkalimetallhydroxid-(z. B. Kaliumhydroxid-)Lösung bei Raumtemperatur vorgenommen, gegebenenfalls unter Zusatz eines inerten Verdünnungsmittels, wie Dioxan, Tetrahydrofuran oder Toluol.

Die Überführung der Säuren der allgemeinen Formel I (R = —H) bzw. der Ausgestaltungen I* und I** in die Salze kann durch direkte alkalische Hydrolyse der Säurederivate I (R = Niederalkyl) erfolgen. Als alkalischer Reaktionspartner wird diejenige anorganische oder organische Base verwendet, deren Salz gewünscht wird. Man erhält die Salze aber auch, indem man die Säuren I (R = —H) mit dem stöchiometrischen Äquivalent an entsprechender Base, z. B. Natriumhydroxid oder Natriumethanolat umsetzt, oder leicht lösliche Salze durch doppelte Umsetzung in schwer lösliche Salze überführt, oder beliebige Salze in pharmakologisch verträgliche Salze überführt.

Die Überführung der Carbonsäuren der allgemeinen Formel I (R = —H) bzw. der Ausgestaltungen I* und I** in die Niederalkylester (R = Niederalkyl) erfolgt in üblicher Weise. Beispielsweise werden sie mit Niederalkanolen in Gegenwart von starken Säuren, wie Schwefelsäure, p-Toluolsulfonsäure, oder von sauren Ionenaustauschern unter Bedingungen, bei denen keine Decarboxylierung stattfindet, oder mit Dialkylsulfaten oder Alkylhalogeniden in Gegenwart von Diazabicycloundecen oder Diazabicyclononen in inerten Lösungsmitteln, wie Benzol, Toluol, Aceton, verestert.

Die Verbindungen der allgemeinen Formel I fallen normalerweise in Form von racemischen Gemischen an, die mittels bekannter Verfahren in die Enantiomeren getrennt werden. Beispielsweise wandelt man mit einem optisch aktiven Spaltungsmittel das Racemat in Diastereomere um, die anschließend durch selektive Kristallisation getrennt und in die entsprechenden optischen Isomeren übergeführt werden. Als optisch aktive Spaltungsmittel dienen z. B. optisch aktive Basen, wie l- und d-l-Phenylethylamin, Cinchonidin oder d-Ephedrin, aus denen Salze der Säuren der allgemeinen Formel I, oder optisch aktive Alkohole, wie Borneol oder Menthol, aus denen Ester der Säuren der allgemeinen Formel I hergestellt werden. Man kann auch racemische Gemische durch Chromatographie über optisch aktive Sorptionsmittel in die optischen Isomeren zerlegen. Alternativ werden zunächst die $\alpha$-Methylencarbonsäuren II mit einem optisch aktiven Spaltungsmittel umgesetzt, z. B. Borneol oder Menthol, die erhaltenen Produkte werden zu den entsprechenden Diastereomerengemischen der Carbonsäureester oxydiert, aus denen dann in üblicher Weise die optischen Isomeren der Säuren I gewonnen werden.

Zur Herstellung der Epoxi-cycloalkylalkancarbonsäuren der Ausgestaltungen I* und I** werden $\alpha$-Methylencarbonsäuren der allgemeinen Formeln II* und II**

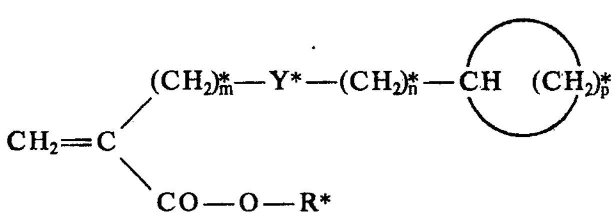

(II*)

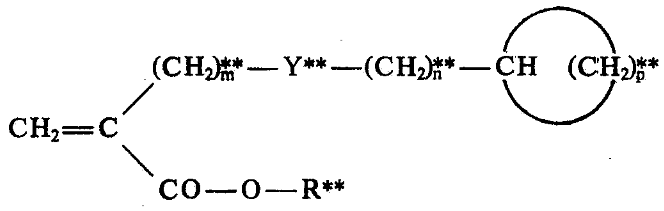

(II**)

worin R*, Y*, m*, n* und p* bzw. R**, Y**, m**, n** und p** die oben angegebene Bedeutung haben, eingesetzt.

Die α-Methylencarbonsäuren der allgemeinen Formeln II, II* und II** können nach an sich bekannten Methoden hergestellt werden. Sie sind wertvolle Zwischenprodukte für die Synthese der Carbonsäuren I, I* und I**.

Die Herstellung der α-Methylencarbonsäuren II erfolgt beispielsweise in Analogie zu H. Stetter und H. Kuhlmann [Synthesis 1979, 29] durch Umsetzung von Malonsäurehalbester der allgemeinen Formel III

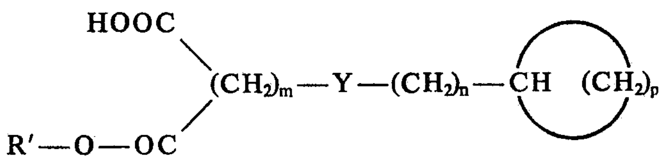

(III)

worin Y, m, n und p die oben angegebene Bedeutung haben und R' eine Niederalkylgruppe bedeutet, mit Formaldehyd in Pyridin in Gegenwart von sekundären Aminen, vorzugsweise Piperidin, und gegebenenfalls anschließende Verseifung der erhaltenen Niederalkylester.

Die Herstellung der Malonsäurehalbester III erfolgt nach Methoden wie sie dem Fachmann geläufig sind, beispielsweise durch Umsetzung von Dialkylmalonaten IV mit Cycloalkylalkylverbindungen V und partielle Verseifung der erhaltenen Malonsäurediester VI nach folgendem Schema

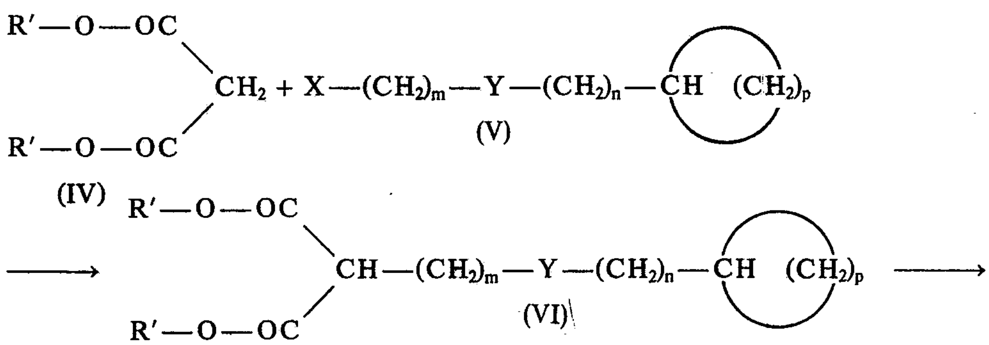

(IV) (V) (VI) (III)

wobei R', Y, m, n und p die oben angegebene Bedeutung haben und X eine Fluchtgruppe, z. B. ein Chlor- oder Bromatom, eine Mesyloxy- oder eine p-Toluolsulfonyloxygruppe, bedeutet.

Für die Herstellung der α-Methylencarbonsäuren II* bzw. II** werden entsprechend substituierte Ausgangsverbindungen III* bzw. III**, IV* bzw. IV**, V* bzw. V**, in denen die Substituenten Y*, m*, n* und p* bzw. Y**, m**, n** und p** die oben angegebenen Bedeutungen haben, R'* eine Niedrigalkylgruppe, R'** eien Methyl- oder Ethylgruppe und X* bzw. X** ein Chlor- oder Bromatom, eine Mesyloxy- oder eine p-Toluolsulfonyloxygruppe bedeuten, eingesetzt.

Die Cycloalkylalkylverbindungen V bzw. deren Ausgestaltungen V* und V** sind bekannte Verbindungen oder werden nach bekannten Verfahren hergestellt.

Beispielsweise können Verbindungen V, in denen Y eine Methylengruppe ($-CH_2-$) darstellt, aus bekannten Ausgangsverbindungen durch übliche Kettenverlängerungsreaktionen hergestellt werden. So lassen sich beispielsweise, ausgehend von Cycloalkylhalogeniden, durch Umsetzung mit Alkalimetallcyaniden oder durch Grignardreaktion mit Kohlendioxid bzw. mit Ethylenoxid, Umwandlung des entstandenen Produktes in die korrespondierende Cycloalkylalkylhalogenverbindung und anschließende weitere, gegebenenfalls mehrfach durchzuführende Kettenverlängerung (z. B. Malonestersynthese mit anschließender Decarboxylierung, Reduktion und Umwandlung des entstandenen Alkohols in die Halogenverbindung) Cycloalkylalkylverbindungen V jeder beliebigen Kettenlänge herstellen.

Verbindungen V, in denen Y ein Sauerstoffatom (—O—) darstellt, können auf verschiedene Weise, z. B. nach folgendem Schema hergestellt werden,

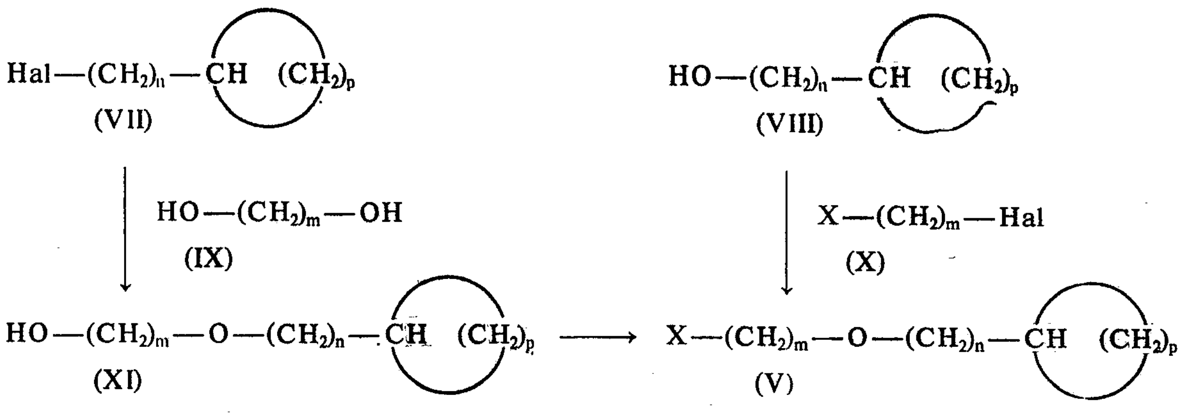


(VII) (VIII) (XI) (V)

worin n, p und X die oben angegebenen Bedeutungen haben, Hal ein Halogenatom und m eine ganze Zahl von 2 bis 6 darstellt [siehe hierzu auch J. Augstein et al. (J. Med. Chem. 8 (1965) 356—367), J. D. Genzer et al. (J. Amer. Chem. Soc. 73 (1951) 3159—3162) und Sh. Mamedov et al., Zh. Obshch. Khim. 32, 799 (1962), 33, 3166 (1963)].

Die Cycloalkylverbindungen VII und VIII sind bekannt oder werden (wie oben beschrieben) in bekannter Weise, z. B. durch Kettenverlängerung hergestellt.

Zur Umsetzung mit den Halogenverbindungen VII bzw. X werden die Alkohole VIII bzw. die Diole IX bevorzugt in die (Mono)alkoholate übergeführt. Die Umwandlung der Alkohole XI in die Verbindungen V, in denen X beispielsweise ein Chloratom darstellt, erfolgt z. B. durch Umsetzung mit Thionylchlorid.

Die folgenden Beispiele dienen zur Erläuterung der Erfindung, ohne sie zu beschränken. Kp bedeutet Siedepunkt, F. bedeutet Schmelzpunkt. Temperaturangaben erfolgen in °C.

## Beispiele

### Beispiel 1

### 2-(3-Cyclohexylpropyl)-oxiran-2-carbonsäureethylester

#### a) 2-(3-Cyclohexylpropyl)-oxiran-2-carbonsäureethylester

7,9 g 5-Cyclohexyl-2-methylenvaleriansäureethylester und 12,2 g m-Chlorperbenzoesäure werden in 100 ml Methylenchlorid 21 Stunden unter Rückfluß gekocht. Man kühlt auf 0° C ab, filtriert den Niederschlag ab, wäscht mit 50 ml Methylenchlorid nach und engt die vereinigten Filtrate ein. Der trübe, ölige Rückstand wird in 60 ml Aceton gelöst und unter Eiskühlung mit verdünnter Sodalösung bis zum Erreichen eines pH-Wertes von 9 versetzt. Man gibt noch 100 ml Wasser zu und extrahiert 3mal mit je 100 ml Diethylether. Die vereinigten organischen Phasen werden nach dem Trocknen über Natriumsulfat eingeengt und der ölige Rückstand destilliert. Man erhält 4,9 g der Titelverbindung vom Kp. 100—108° C bei 0,02 Torr (2,7 Pa).

#### b) 5-Cyclohexyl-2-methylenvaleriansäureethylester

51 g (3-Cyclohexylpropyl)-malonsäureethylester, 6,4 g Paraformaldehyd, 50 ml Pyridin und 2,1 g Piperidin werden auf 60° C erwärmt. Nach Beendigung der Kohlendioxidentwicklung (ca. 1 Stunde) rührt man noch eine Stunde bei 60° C, gießt in 800 ml Wasser und extrahiert 4mal mit je 200 ml Petrolether. Die vereinigten organischen Phasen werden mit je 200 ml Wasser, 2n Salzsäure, Wasser und gesättigter Natriumhydrogencarbonatlösung gewaschen, über Natriumsulfat getrocknet und eingeengt. Der Rückstand wird durch Chromatographie an einer Kieselgelsäule gereinigt. (Laufmittel Petrolether/Essigsäureethylester 80 : 20). Man erhält 36,7 g 5-Cyclohexyl-2-methylenvaleriansäureethylester als fast farbloses Öl.

#### c) (3-Cyclohexylpropyl)-malonsäureethylester

Zu 68 g (3-Cyclohexylpropyl)-malonsäurediethylester (hergestellt analog Beispiel 4d), gelöst in 160 ml Ethanol, tropft man unter Rühren während einer Stunde eine Lösung von 13,5 g Kaliumhydroxid in 160 ml Ethanol. Nach 24 Stunden Rühren bei Raumtemperatur wird im Vakuum weitgehend einge-

engt, der Rückstand in 400 ml Wasser gelöst und mit 300 ml Diethylether extrahiert. Die wäßrige Phase wird mit 40 ml halbkonz. Salzsäure unter Eiskühlung versetzt und 3mal mit je 300 ml Diethylether extrahiert. Die vereinigten organischen Phasen werden über Natriumsulfat getrocknet und eingeengt. Man erhält 51 g (3-Cyclohexylpropyl)-malonsäureethylester als zähes, gelbliches Öl.

Beispiel 2

2-(4-Cyclohexylbutyl)-oxiran-2-carbonsäureethylester

a) 2-(4-Cyclohexylbutyl)-oxiran-2-carbonsäureethylester

Nach der in Beispiel 1a) beschriebenen Arbeitsweise erhält man aus 6,4 g 6-Cyclohexyl-2-methylenhexansäureethylester und 9,3 g m-Chlorperbenzoesäure in 80 ml Methylenchlorid 3,8 g der Titelverbindung als farbloses Öl vom Kp. 110—118° C bei 0,01 Torr (1,3 Pa).

b) 6-Cyclohexyl-2-methylenhexansäureethylester

Nach der in Beispiel 1b) beschriebenen Arbeitsweise erhält man aus 26 g (4-Cyclohexylbutyl)-malonsäureethylester, 3,1 g Paraformaldehyd, 19 ml Pyridin und 1,2 ml Piperidin 16,6 g 6-Cyclohexyl-2-methylenhexansäureethylester als fast farbloses Öl, gereinigt durch Chromatographie an Kieselgel (Laufmittel: Petrolether/Essigsäureethylester 80 : 20).

c) (4-Cyclohexylbutyl)-malonsäureethylester

Nach der in Beispiel 1c) beschriebenen Arbeitsweise erhält man aus 35 g (4-Cyclohexylbutyl)-malonsäurediethylester (hergestellt analog Beispiel 4d) und 6,7 g Kaliumhydroxid in 150 ml Ethanol 26,1 g (4-Cyclohexylbutyl)-malonsäureethylester als zähes, gelbliches Öl.

Beispiel 3

2-(5-Cyclohexylpentyl)-oxiran-2-carbonsäureethylester

a) 2-(5-Cyclohexylpentyl)-oxiran-2-carbonsäureethylester

Nach der in Beispiel 1a) beschriebenen Arbeitsweise erhält man aus 9,9 g 7-Cyclohexyl-2-methylenheptansäureethylester und 13,6 g m-Chlorperbenzoesäure in 100 ml Methylenchlorid 5,3 g der Titelverbindung vom Kp. 115—123° C bei 0,005 Torr (0,7 Pa).

b) 7-Cyclohexyl-2-methylenheptansäureethylester

Analog Beispiel 1b) erhält man aus 35,5 g (5-Cyclohexylpentyl)-malonsäureethylester, 4,0 g Paraformaldehyd, 25 ml Pyridin und 1,5 ml Piperidin nach Chromatographie (Petrolether/Methylenchlorid 1 : 1) 23,6 g 7-Cyclohexyl-2-methylenheptansäureethylester als farbloses Öl.

c) (5-Cyclohexylpentyl)-malonsäureethylester

Nach der in Beispiel 1c) beschriebenen Arbeitsweise erhält man aus 47,2 g (5-Cyclohexylpentyl)-malonsäurediethylester (hergestellt analog Beispiel 4d) und 8,6 g Kaliumhydroxid in 200 ml Ethanol 35,5 g (5-Cyclohexylpentyl)-malonsäureethylester als zähes, gelbliches Öl.

## Beispiel 4

### 2-[4-(Cyclohexyloxy)-butyl]-oxiran-2-carbonsäureethylester

a) 2-[4-(Cyclohexyloxy)-butyl]-oxiran-2-carbonsäureethylester

Nach der in Beispiel 1a) beschriebenen Arbeitsweise erhält man aus 4,9 g 6-Cyclohexyloxy-2-methylenhexansäureethylester und 7 g m-Chlorperbenzoesäure in 50 ml Methylenchlorid 2,4 g der Titelverbindung als farbloses Öl, gereinigt durch Chromatographie an Kieselgel (Laufmittel: Petrolether/Essigsäureethylester 90 : 10).

b) 6-Cyclohexyloxy-2-methylenhexansäureethylester

Nach der in Beispiel 1b) beschriebenen Arbeitsweise erhält man aus 7,4 g [4-(Cyclohexyloxy)-butyl]-malonsäureethylester, 0,85 g Paraformaldehyd, 5,2 ml Pyridin und 0,3 ml Piperidin 4,9 g 6-Cyclohexyloxy-2-methylenhexansäureethylester als farbloses Öl, gereinigt durch Chromatographie an Kieselgel (Laufmittel: Petrolether/Essigsäureethylester 90 : 10).

c) [4-(Cyclohexyloxy)-butyl]-malonsäureethylester

Nach der in Beispiel 1c) beschriebenen Arbeitsweise erhält man aus 10,5 g [4-(Cyclohexyloxy)-butyl]-malonsäurediethylester und 1,9 g Kaliumhydroxid in 50 ml Ethanol 7,4 g [4-(Cyclohexyloxy)-butyl]-malonsäureethylester.

d) [4-(Cyclohexyloxy)-butyl]-malonsäurediethylester

Man tropft bei 50° C 15 g Malonsäurediethylester zu einer aus 1,5 g Natrium und 30 ml Ethanol frisch hergestellten Natriumethylatlösung. Man hält 1 Stunde bei dieser Temperatur und tropft anschließend 11,7 g 4-Cyclohexyloxybutylbromid zu. Nach beendeter Zugabe rührt man 3 Stunden bei 60° C, engt weitgehend ein, versetzt den Rückstand mit 100 ml Eiswasser und extrahiert 3mal mit insgesamt 150 ml Methylenchlorid. Die vereinigten organischen Phasen werden über Natriumsulfat getrocknet und das Lösungsmittel sowie überschüssiger Malonsäurediethylester im Vakuum abdestilliert. Man erhält 10,5 g [4-(Cyclohexyloxy)-butyl]-malonsäurediethylester als hellgelben öligen Rückstand.

## Beispiel 5

### 2-[4-(2-Cyclohexylethoxy)-butyl]-oxiran-2-carbonsäureethylester

a) 2-[4-(2-Cyclohexylethoxy)-butyl]-oxiran-2-carbonsäureethylester

Nach der in Beispiel 1a) beschriebenen Arbeitsweise erhält man aus 11,2 g 6-(2-Cyclohexylethoxy)-2-methylenhexansäureethylester und 13,7 g m-Chlorperbenzoesäure in 100 ml Methylenchlorid 5,3 g der Titelverbindung als Öl, gereinigt durch Chromatographie an Kieselgel (Laufmittel: Petrolether/Essigsäureethylester 9 : 1).

b) 6-(2-Cyclohexylethoxy)-2-methylenhexansäureethylester

Nach der in Beispiel 1b beschriebenen Arbeitsweise erhält man aus 16,5 g [4-(2-Cyclohexylethoxy)-butyl]-malonsäureethylester, 1,97 g Paraformaldehyd, 12 ml Pyridin und 0,7 ml Piperidin 11,2 g 6-(2-Cyclohexylethoxy)-2-methylenhexansäureethylester als fast farbloses Öl, gereinigt durch Chromatographie an Kieselgel (Laufmittel: Petrolether/Essigsäureethylester 9 : 1).

c) [4-(2-Cyclohexylethoxy)-butyl]-malonsäureethylester

Nach der in Beispiel 1c) beschriebenen Arbeitsweise erhält man aus 22 g [4-(2-Cyclohexylethoxy)-butyl]-malonsäurediethylester und 3,6 g Kaliumhydroxid in 150 ml Ethanol 16,5 g [4-(2-Cyclohexylethoxy)-butyl]-malonsäureethylester als dickes, gelbes Öl.

d) [4-(2-Cyclohexylethoxy)-butyl]-malonsäurediethylester

Nach der in Beispiel 4d) beschriebenen Arbeitsweise erhält man aus 27,7 g 4-(2-Cyclohexylethoxy)-butylbromid, 25,3 g Malonsäurediethylester und einer Lösung aus 2,9 g Natrium in 150 ml Ethanol 22 g [4-(2-Cyclohexylethoxy)-butyl]-malonsäurediethylester als gelbes Öl.

Beispiel 6

2-(7-Cyclohexylheptyl)-oxiran-2-carbonsäureethylester

a) 2-(7-Cyclohexylheptyl)-oxiran-2-carbonsäureethylester

Nach der in Beispiel 1a) beschriebenen Arbeitsweise erhält man aus 9,2 g 9-Cyclohexyl-2-methylennonansäureethylester und 11,3 g m-Chlorperbenzoesäure in 100 ml Methylenchlorid 5,25 g der Titelverbindung als farbloses Öl, gereinigt durch Chromatographie an Kieselgel (Laufmittel: Petrolether/Essigsäureethylester 90 : 10).

b) 9-Cyclohexyl-2-methylennonansäureethylester

Nach der in Beispiel 1b) beschriebenen Arbeitsweise erhält man aus 16,2 g (7-Cyclohexylheptyl)-malonsäureethylester, 1,86 g Paraformaldehyd, 11,5 ml Pyridin und 0,6 ml Piperidin 9,2 g 9-Cyclohexyl-2-methylennonansäureethylester als gelbliches Öl, gereinigt durch Chromatographie an Kieselgel (Laufmittel: Petrolether/Essigsäureethylester 95 : 5).

c) (7-Cyclohexylheptyl)-malonsäureethylester

Nach der in Beispiel 1c) beschriebenen Arbeitsweise erhält man aus 21,6 g (7-Cyclohexylheptyl)-malonsäurediethylester (hergestellt analog Beispiel 4d) und 3,55 g Kaliumhydroxid in 150 ml Ethanol 16,3 g (7-Cyclohexylheptyl)-malonsäureethylester als gelbes, dickes Öl.

Beispiel 7

2-(6-Cyclopropylhexyl)-oxiran-2-carbonsäureethylester

a) 2-(6-Cyclopropylhexyl)-oxiran-2-carbonsäureethylester

Nach der in Beispiel 1a) beschriebenen Arbeitsweise erhält man aus 2,5 g 8-Cyclopropyl-2-methylenoctansäureethylester und 3,85 g m-Chlorperbenzoesäure in 30 ml Methylenchlorid 1,18 g der Titelverbindung als gelbliches Öl, gereinigt durch Chromatographie an Kieselgel (Laufmittel: Petrolether/Essigsäureethylester 90 : 10).

b) 8-Cyclopropyl-2-methylenoctansäureethylester

Nach der in Beispiel 1b) beschriebenen Arbeitsweise erhält man aus 4,53 g (6-Cyclopropylhexyl)-malonsäureethylester, 0,64 g Paraformaldehyd, 4 ml Pyridin und 0,2 ml Piperidin 2,5 g 8-Cyclopropyl-2-methylenoctansäureethylester als gelbliches Öl, gereinigt durch Chromatographie an Kieselgel (Laufmittel: Petrolether/Essigsäureethylester 90 : 10).

c) (6-Cyclopropylhexyl)-malonsäureethylester

Nach der in Beispiel 1c) beschriebenen Arbeitsweise erhält man aus 6,4 g (6-Cyclopropylhexyl)-malonsäurediethylester und 1,26 g Kaliumhydroxid in 40 ml Ethanol 4,53 g (6-Cyclopropylhexyl)-malonsäureethylester als gelbes Öl.

d) (6-Cyclopropylhexyl)-malonsäurediethylester

Nach der in Beispiel 4d) beschriebenen Arbeitsweise erhält man aus 6,8 g 6-Cyclopropylhexylbromid, 7,95 g Malonsäurediethylester und einer Lösung aus 0,91 g Natrium in 40 ml Ethanol 6,4 g (6-Cyclopropylhexyl)-malonsäurediethylester als gelbes Öl.

Beispiel 8

2-(3-Cyclopentylpropyl)-oxiran-2-carbonsäureethylester

a) 2-(3-Cyclopentylpropyl)-oxiran-2-carbonsäureethylester

Nach der in Beispiel 1a) beschriebenen Arbeitsweise erhält man aus 20 g 5-Cyclopentyl-2-methylenvaleriansäureethylester und 24,2 g m-Chlorperbenzoesäure in 250 ml Methylenchlorid 19 g der Titelverbindung als fabloses Öl vom Kp. 89—93° C bei 0,04 Torr (5,3 Pa).

b) 5-Cyclopentyl-2-methylenvaleriansäureethylester

Nach der in Beispiel 1b) beschriebenen Arbeitsweise erhält man aus 43,8 g (3-Cyclopentylpropyl)-malonsäureethylester, 5,7 g Paraformaldehyd, 45 ml Pyridin und 1,55 g Piperidin 20,2 g 5-Cyclopentyl-2-methylenvaleriansäureethylester vom Kp. 63—65° C bei 0,01 Torr (1,3 Pa).

c) (3-Cyclopentylpropyl)-malonsäureethylester

Nach der in Beispiel 1c) beschriebenen Arbeitsweise erhält man aus 72,8 g (3-Cyclopentylpropyl)-malonsäurediethylester (hergestellt analog Beispiel 4d) und 17,7 g Kaliumhydroxid in 580 ml Ethanol 43,8 g (3-Cyclopentylpropyl)-malonsäureethylester als gelbes Öl.

Beispiel 9

2-(4-Cyclopentylbutyl)-oxiran-2-carbonsäureethylester

a) 2-(4-Cyclopentylbutyl)-oxiran-2-carbonsäureethylester

Nach der in Beispiel 1a) beschriebenen Arbeitsweise erhält man aus 8,5 g 6-Cyclopentyl-2-methylenhexansäureethylester und 13,1 g m-Chlorperbenzoesäure in 120 ml Methylenchlorid 4,0 g der Titelverbindung als farbloses Öl vom Kp. 100—107° C bei 0,03 Torr (4 Pa).

b) 6-Cyclopentyl-2-methylenhexansäureethylester

Nach der in Beispiel 1b) beschriebenen Arbeitsweise erhält man aus 18,3 g (4-Cyclopentylbutyl)-malonsäureethylester, 2,6 g Paraformaldehyd, 20 ml Pyridin und 0,9 ml Piperidin 8,5 g 6-Cyclopentyl-2-methylenhexansäureethylester vom Kp. 80—87° C bei 0,01 Torr (1,3 Pa).

c) (4-Cyclopentylbutyl)-malonsäureethylester

Nach der in Beispiel 1c) beschriebenen Arbeitsweise erhält man aus 29,9 g (4-Cyclopentylbutyl)-malonsäurediethylester (hergestellt analog Beispiel 4d) und 5,9 g Kaliumhydroxid in 200 ml Ethanol 18,3 g (4-Cyclopentylbutyl)-malonsäureethylester als zähes Öl.

Beispiel 10

2-(6-Cyclopentylhexyl)-oxiran-2-carbonsäureethylester

a) 2-(6-Cyclopentylhexyl)-oxiran-2-carbonsäureethylester

Nach der in Beispiel 1a) beschriebenen Arbeitsweise erhält man aus 11,4 g 8-Cyclopentyl-2-methylenoctansäureethylester und 15,6 g m-Chlorperbenzoesäure in 120 ml Methylenchlorid 5,33 g der Titelverbindung als fast farbloses Öl, gereinigt durch Chromatographie an Kieselgel (Laufmittel: Petrolether/Essigsäureethylester 90 : 10).

b) 8-Cyclopentyl-2-methylenoctansäureethylester

Nach der in Beispiel 1b) beschriebenen Arbeitsweise erhält man aus 22,1 g (6-Cyclopentylhexyl)-malonsäureethylester, 2,8 g Paraformaldehyd, 22 ml Pyridin und 1 ml Piperidin 11,5 g 8-Cyclopentyl-2-methylenoctansäureethylester als fast farbloses Öl, gereinigt durch Chromatographie an Kieselgel (Laufmittel: Petrolether/Essigsäureethylester 95 : 5).

c) (6-Cyclopentylhexyl)-malonsäureethylester

Nach der in Beispiel 1c) beschriebenen Arbeitsweise erhält man aus 37,9 g (6-Cyclopentylhexyl)-malonsäurediethylester und 6,8 g Kaliumhydroxid in 200 ml Ethanol 22,1 g (6-Cyclopentylhexyl)-malonsäureethylester als gelbes Öl.

d) (6-Cyclopentylhexyl)-malonsäurediethylester

Nach der in Beispiel 4d) beschriebenen Arbeitsweise erhält man aus 48,9 g 6-Cyclopentylhexylbromid, 50,3 g Malonsäurediethylester und einer Lösung von 5,8 g Natrium in 300 ml Ethanol 38 g (6-Cyclopentylhexyl)-malonsäurediethylester als Öl.

Beispiel 11

2-(3-Cycloheptylpropyl)-oxiran-2-carbonsäureethylester

a) 2-(3-Cycloheptylpropyl)-oxiran-2-carbonsäureethylester

Nach der in Beispiel 1a) beschriebenen Arbeitsweise erhält man aus 18,2 g 5-Cycloheptyl-2-methylenvaleriansäureethylester und 26,3 g m-Chlorperbenzoesäure in 200 ml Methylenchlorid 9,3 g der Titelverbindung vom Kp. 107—110° C bei 0,08 Torr (10,6 Pa).

b) 5-Cycloheptyl-2-methylenvaleriansäureethylester

Nach der in Beispiel 1b) beschriebenen Arbeitsweise erhält man aus 33,3 g (3-Cycloheptylpropyl)-malonsäureethylester, 4,43 g Paraformaldehyd, 35 ml Pyridin und 1,6 ml Piperidin 18,2 g 5-Cycloheptyl-2-methylenvaleriansäureethylester vom Kp. 89—98° C bei 0,01 Torr (1,3 Pa).

c) (3-Cycloheptylpropyl)-malonsäureethylester

Nach der in Beispiel 1c) beschriebenen Arbeitsweise erhält man aus 49,7 g (3-Cycloheptylpropyl)-malonsäurediethylester und 9,33 g Kaliumhydroxid in 300 ml Ethanol 33,3 g (3-Cycloheptylpropyl)-malonsäureethylester als gelbes Öl.

d) (3-Cycloheptylpropyl)-malonsäurediethylester

Nach der in Beispiel 4d) beschriebenen Arbeitsweise erhält man aus 63 g 3-Cycloheptylpropylbromid, 69 g Malonsäurediethylester und einer Lösung aus 7,9 g Natrium in 400 ml Ethanol 49,8 g (3-Cycloheptylpropyl)-malonsäurediethylester vom Kp. 120—122° C/0,08 Torr (10,6 Pa).

Beispiel 12

2-(3-Cyclooctylpropyl)-oxiran-2-carbonsäureethylester

a) 2-(3-Cyclooctylpropyl)-oxiran-2-carbonsäureethylester

Nach der in Beispiel 1a) beschriebenen Arbeitsweise erhält man aus 5,42 g 5-Cyclooctyl-2-methylenvaleriansäureethylester und 5,55 g m-Chlorperbenzoesäure in 50 ml Methylenchlorid 2,2 g der Titelverbindung als farbloses Öl, gereinigt durch Cromatographie an Kieselgel (Laufmittel: Petrolether/Essigsäureethylester 90 : 10).

b) 5-Cyclooctyl-2-methylenvaleriansäureethylester

Nach der in Beispiel 1b) beschriebenen Arbeitsweise erhält man aus 10,1 g (3-Cyclooctylpropyl)-malonmsäureethylester, 1,28 g Paraformaldehyd, 10 ml Pyridin und 0,5 ml Piperidin 5,5 g 5-Cyclooctyl-2-methylenvaleriansäureethylester als Öl, gereinigt durch Chromatographie an Kieselgel (Laufmittel: Petrolether/Essigsäureethylester 95 : 5).

c) (3-Cyclooctylpropyl)-malonsäureethylester

Nach der in Beispiel 1c) beschriebenen Arbeitsweise erhält man aus 16 g (3-Cyclooctylpropyl)-malonsäurediethylester und 2,87 g Kaliumhydroxid 10,2 g (3-Cyclooctylpropyl)-malonsäureethylester als gelbliches Öl.

d) (3-Cyclooctylpropyl)-malonsäurediethylester

Nach der in Beispiel 4d) beschriebenen Arbeitsweise erhält man aus 20,0 g 3-Cyclooctylpropylbromid, 20,6 g Malonsäurediethylester und einer Lösung aus 2,37 g Natrium in 180 ml Ethanol 16 g (3-Cyclooctylpropyl)-malonsäurediethylester als gelbliches Öl.

e) 3-Cyclooctylpropylbromid

19,7 g 2-Cyclooctylpropanol-1 werden mit 35 ml 48%iger Bromwasserstoffsäure und 0,1 g rotem Phosphor 3 Stunden unter Rückfluß gekocht, 7 ml konzentrierte Schwefelsäure zugegeben und weitere 3 Stunden gekocht. Man gießt in 100 ml Eiswasser, extrahiert 2mal mit je 100 ml Diethylether, trocknet die vereinigten organischen Phasen über Natriumsulfat und engt ein. Zurück bleiben 20,0 g 3-Cyclooctylpropylbromid als braunes Öl.

Beispiel 13

2 (3 Cycloundecylpropyl) oxiran 2 carbonsäureethylester

a) 2-(3-Cycloundecylpropyl)-oxiran-2-carbonsäureethylester

Nach der in Beispiel 1a) beschriebenen Arbeitsweise erhält man aus 4,4 g 5-Cycloundecyl-2-methylenvaleriansäureethylester und 5,15 g m-Chlorperbenzoesäure in 40 ml Methylenchlorid 2,2 g der Titelverbindung als farbloses Öl, gereinigt durch Chromatographie an Kieselgel (Laufmittel: Petrolether/Essigsäureethylester 90 : 10). Dünnschichtchromatographie an Kieselgel mit Petrolether/Essigsäureethylester 9 : 1 : $R_f = 0,5$.

b) 5-Cycloundecyl-2-methylenvaleriansäureethylester

Nach der in Beispiel 1b) beschriebenen Arbeitsweise erhält man aus 7,6 g (3-Cycloundecylpropyl)-malonsäureethylester, 0,84 g Paraformaldehyd, 7 ml Pyridin und 0,3 ml Piperidin 4,4 g 5-Cycloundecyl-2-methylenvaleriansäureethylester als gelbliches Öl, gereinigt durch Chromatographie an Kieselgel (Laufmittel: Petrolether/Essigsäureethylester 95 : 5).

c) (3-Cycloundecylpropyl)-malonsäureethylester

Nach der in Beispiel 1c) beschriebenen Arbeitsweise erhält man aus 10,6 g (3-Cycloundecylpropyl)-malonsäurediethylester und 1,67 g Kaliumhydroxid in 40 ml Ethanol 7,6 g (3-Cycloundecylpropyl)-malonsäureethylester als dickes, gelbes Öl.

d) (3-Cycloundecylpropyl)-malonsäurediethylester

Nach der in Beispiel 4d) beschriebenen Arbeitsweise erhält man aus 15 g 3-Cycloundecylpropylbromid, 13 g Malonsäurediethylester und einer Lösung aus 1,5 g Natrium in 80 ml Ethanol 10,6 g (3-Cycloundecylpropyl)-malonsäurediethylester als Öl.

e) 3-Cycloundecylpropylbromid

Nach der in Beispiel 12e) beschriebenen Arbeitsweise erhält man aus 16 g 3-Cycloundecylpropanol-1, 30 ml 48%iger Bromwasserstoffsäure, 0,1 g rotem Phosphor und 5 ml konzentrierter Schwefelsäure 15 g 3-Cycloundecylpropylbromid als Öl.

Das Ausgangsmaterial 3-Cycloundecylpropanol-1 erhält man wie folgt: Hydroxymethylcycloundecan wird mit Thionylchlorid zum Chlormethylcycloundecan umgesetzt, das mit Malonsäurediethylester in Gegenwart von Natriumethanolat zum (Cycloundecylmethyl)-malonsäurediethylester umgesetzt wird, der nach Verseifung der Estergruppe und Decarboxylierung der entsprechenden Malonsäure 3-Cycloundecylpropionsäure ergibt. 3-Cycloundecylpropionsäure wird mit Lithiumaluminiumhydrid in Tetrahydrofuran zum 3-Cycloundecylpropanol reduziert.

Beispiel 14

Natrium-2-(3-cyclohexylpropyl)-oxiran-2-carboxylat

1,1 g 2-(3-Cyclohexylpropyl)-oxiran-2-carbonsäureethylester werden mit 4,58 ml in Natronlauge und 5 ml Tetrahydrofuran 2 Stunden bei Raumtemperatur gerührt. Man engt auf die Hälfte des Volumens ein und filtriert die ausgefallenen Kristalle der Titelverbindung ab. F. 105 – 110° C.

Beispiel 15

2-[2-(3-Cyclohexylpropyloxy)-ethyl]-oxiran-2-carbonsäureethylester

a) 2-[2-(3-Cyclohexylpropyloxy)-ethyl]-oxiran-2-carbonsäureethylester

Nach der in Beispiel 1a) beschriebenen Arbeitsweise erhält man aus 12,4 g 4-(3-Cyclohexylpropyloxy)-2-methylenbuttersäure-ethylester und 18,7 g m-Chlorperbenzoesäure in 250 ml Methylenchlorid 6,8 g der Titelverbindung als farbloses Öl, gereinigt durch Chromatographie an Kieselgel (Laufmittel Petrolether/Essigsäureethylester 9 : 1). Dünnschichtchromatographie an Kieselgel mit Petrolether/Essigsäureethylester/Eisessig 80 : 20 : 3 : $R_f = 0{,}65$.

b) 4-(3-Cyclohexylpropyloxy)-2-methylenbuttersäureethylester

Nach der in Beispiel 1b) beschriebenen Arbeitsweise erhält man aus 103,6 g 2-(3-Cyclohexylpropyloxy)-ethylmalonsäureethylester, 11 g Paraformaldehyd, 105 ml Pyridin und 2,9 g Piperidin 82,3 g 4-(3-Cyclohexylpropyloxy)-2-methylenbuttersäureethylester als farbloses Öl, gereinigt durch Chromatographie an Kieselgel (Laufmittel Chloroform)

c) 2-(3-Cyclohexylpropyloxy)-ethylmalonsäureethylester

Nach der in Beispiel 1c) beschriebenen Arbeitsweise erhält man aus 125,4 g 2-(3-Cyclohexylpropyloxy)-ethylmalonsäurediethylester und 24,5 g Kaliumhydroxid in 750 ml Ethanol 104,6 g 2-(3-Cyclohexylpropyloxy)-ethylmalonsäureethylester als gelbliches Öl.

d) 2-(3-Cyclohexylpropyloxy)-ethylmalonsäurediethylester

Nach der in Beispiel 4d) beschriebenen Arbeitsweise erhält man aus 118,6 g 2-(3-Cyclohexylpropyloxy)-ethylchlorid, 97,4 g Malonsäurediethylester, einer Lösung aus 13,3 g Natrium in 420 ml Ethanol und 0,5 g Kaliumiodid 125,9 g 2-(3-Cyclohexylpropyloxy)-ethylmalonsäurediethylester als farbloses Öl vom Kp. 138° – 140° C bei 0,03 Torr (4,0 Pa).

e) 2-(3-Cylohexylpropyloxy)-ethylchlorid

110,4 g 2-(3-Cyclohexylpropyloxy)-ethanol, 2 ml Pyridin und 112 ml Thionylchlorid werden 30 Minuten unter Rückfluß gekocht. Nach dem Abkühlen wird die Reaktionsmischung auf 500 g Eis gegossen, mit Methylenchlorid (2 × 500 ml) extrahiert, die vereinigten organischen Phasen nacheinander mit Wasser, Natriumhydrogencarbonat und Wasser gewaschen, eingeengt und der Rückstand destilliert. Man erhält 119,6 g 2-(3-Cyclohexylpropyloxy)-ethylchlorid vom Kp. 68° – 69° C bei 0,01 Torr (1,3 Pa).

f) 2-(3-Cyclohexylpropyloxy)-ethanol

Zu einer Lösung von 15,75 g Natrium in 182,2 g Ethylenglykol gibt man 85 ml Xylol, erwärmt auf 100° C und tropft innerhalb 3 Stunden eine Lösung von 140,5 g 3-Cyclohexylpropylbromid in 70 ml Xylol zu. Nach beendeter Zugabe kocht man 4 Stunden unter Rückfluß, läßt abkühlen und versetzt mit 500 ml Wasser. Man extrahiert mit Petrolether (2 x 500 ml), trocknet die vereinigten organischen Phasen über Natriumsulfat, engt ein und reinigt das zurückbleibende Öl durch Chromatographie an Kieselgel (Laufmittel Petrolether/Essigsäureethylester 8 : 2). Man erhält 105,3 g 2-(3-Cyclohexylpropyloxy)-ethanol.

Beispiel 16

2-(5-Cycloheptylpentyl)-oxiran-2-carbonsäureethylester

a) 2-(5-Cycloheptylpentyl)-oxiran-2-carbonsäureethylester

Nach der in Beispiel 1a) beschriebenen Arbeitsweise erhält man aus 13,1 g 7-Cycloheptyl-2-methylenheptansäureethylester und 12,0 g m-Chlorperbenzoesäure in 200 ml Methylenchlorid 7,5 g der Titelverbindung als farbloses Öl, gereinigt durch Chromatographie an Kieselgel (Laufmittel Petrolether/Essigsäureethylester 9 : 1). Dünnschichtchromatographie an Kieselgel mit Petrolether/Essigsäureethylester 8 : 2 : $R_f = 0,55$.

b) 7-Cycloheptyl-2-methylenheptansäureethylester

Nach der in Beispiel 1b) beschriebenen Arbeitsweise erhält man aus 35 g 5-Cycloheptylpentylmalonsäureethylester, 3,8 g Paraformaldehyd, 35 ml Pyridin und 1 g Piperidin 23,7 g 7-Cycloheptyl-2-methylenheptansäureethylester als farbloses Öl, gereinigt durch Chromatographie an Kieselgel (Laufmittel: Petrolether/Essigsäureethylester 9 : 1).

c) 5-Cycloheptylpentylmalonsäureethylester

Nach der in Beispiel 1c) beschriebenen Arbeitsweise erhält man aus 55,7 g 5-Cycloheptylpentylmalonsäurediethylester und 11,3 g Kaliumhydroxid in 220 ml Ethanol 35,8 g 5-Cycloheptylpentylmalonsäureethylester als zähes Öl.

d) 5-Cycloheptylpentylmalonsäurediethylester

Nach der in Beispiel 4d) beschriebenen Arbeitsweise erhält man aus 44,8 g 5-Cycloheptylpentylbromid, 32 g Malonsäurediethylester und einer Lösung von 4,2 g Natrium in 140 ml Ethanol 55,7 g der Titelverbindung als fast farbloses Öl.

e) 5-Cycloheptylpentylbromid

Nach der in Beispiel 12e) beschriebenen Arbeitsweise erhält man aus 35,3 g 5-Cycloheptylpentanol-1, 57 ml 48% Bromwasserstoffsäure, 0,2 g rotem Phosphor und 12 ml konz. Schwefelsäure 44,9 g 5-Cycloheptylpentylbromid als hellgelbes Öl vom Kp. 135° —138° C bei 12 Torr (1600 Pa).

Das Ausgangsmaterial 5-Cycloheptylpentanol-1 erhält man wie folgt: Cycloheptylpropylmalonsäureethylester wird 1 Stunde auf 180° C erhitzt und der resultierende 5-Cycloheptylvaleriansäureethylester [Kp. 80° —82° C bei 0,008 Torr (1,0 Pa)] mit Lithiumaluminiumhydrid in Tetrahydrofuran zu 5-Cycloheptylpentanol-1 [Kp. 92° —94° C bei 0,008 Torr (1,0 Pa)] reduziert.

Beispiel 17

Natrium-2-(5-cyclohexylpentyl)-oxiran-2-carboxylat

1,5 g 2-(5-cyclohexylpentyl)-oxiran-2-carbonsäureethylester werden mit 5,6 ml 1n Natronlauge und 5 ml Tetrahydrofuran 2 Stunden bei Raumtemperatur gerührt. Man engt auf die Hälfte des Volumens ein und filtriert die ausgefallenen fettig glänzenden Plättchen der Titelverbindung ab.

## Galenische Beispiele

### Beispiel 1

Ansatz für Ampullen

100 g Natrium-2-(5-cyclohexylpentyl)-oxiran-2-carboxylat werden in ca. 8 Liter aqua bidest. unter Zusatz der äquivalenten Menge Natronlauge gelöst. Die Lösung wird auf pH 7,0±0,5 eingestellt und mit aqua bidest. auf 10 Liter aufgefüllt. Dann wird steril filtriert und unter keimfreien Bedingungen in 2 ml-Ampullen abgefüllt.

### Beispiel 2

10 000 Kapseln mit einem Wirkstoffgehalt von 30 mg werden aus folgenden Bestandteilen hergestellt:
300 g 2-[2-(3-Cyclohexylpropyloxy)-ethyl]-oxiran-2-carbonsäureethylester werden mit 500 g Neutralöl gemischt und in Weichgelatinekapseln abgefüllt.

### Beispiel 3

Tabletten mit einem Wirkstoffgehalt von 25 mg werden wie folgt hergestellt:
1,0 kg Natrium-2-(3-cyclohexylpropyl)-oxiran-2-carboxylat, 4,5 kg Xylit und 3,0 kg Calciumphosphat werden mit 0,25 kg Polyvinylpyrrolidon (MG 25 000; MG = Molekulargewicht) in ungefähr 0,5 l Wasser granuliert. Das Granulat wird durch ein Sieb von 1,25 mm Maschenweite gesiebt und nach dem Trocknen werden 0,9 kg Carboxymethylcellulose, 0,25 kg Talkum und 0,1 kg Magnesiumstearat zugegeben. Man verpreßt das trockene Granulat zu Tabletten von 8 mm Durchmesser, 250 mg Gewicht und einer Härte von 5—6 kg.

## Pharmakologie

Die erfindungsgemäßen Epoxi-cycloalkylalkancarbonsäuren der allgemeinen Formel I senken den Blutglucosespiegel und den Blutspiegel der Ketonkörper, wobei sie sich in ihrer chemischen Struktur und ihrer Wirkungsweise grundlegend von pankreaswirksamen, betacytotropen Substanzen (z. B. Sulfonylharnstoffen) durch ihre extrapankreatische Wirkung unterscheiden und extrapankreatisch wirkenden Handelspräparaten (z. B. Buformin und Phenformin) überlegen erweisen.

In der anschließenden Tabelle werden die untersuchten Verbindungen durch eine laufende Nummer gekennzeichnet, die wie folgt zuzuordnen ist:

| Lfd. Nr. | Name der Verbindung |
|---|---|
| 1 | Buformin |
| 2 | Phenformin |
| 3 | 2-(3-Cyclohexylpropyl)-oxiran-2-carbonsäureethylester |
| 4 | 2-(5-Cyclohexylpentyl)-oxiran-2-carbonsäureethylester |
| 5 | 2-(3-Cyclopentylpropyl)-oxiran-2-carbonsäureethylester |
| 6 | 2-(4-Cyclopentylbutyl)-oxiran-2-carbonsäureethylester |
| 7 | 2-[2-(3-Cyclohexylpropyloxy)-ethyl]-oxiran-2-carbonsäureethylester |
| 8 | 2-(3-Cycloheptylpropyl)-oxiran-2-carbonsäureethylester |
| 9 | 2-(5-Cycloheptylpentyl)-oxiran-2-carbonsäureethylester |

In Tabelle I werden Untersuchungen des Einflusses von Vertretern der erfindungsgemäßen Verbindungen auf die Blutglucosekonzentration nüchterner, stoffwechselgesunder Ratten wiedergegeben. Spalte A gibt jeweils die maximale Senkung der Blutglucosekonzentration nüchterner Ratten (in %) bezogen auf die Kontrollgruppe) an, die im Laufe von 6 Stunden nach einmaliger oraler Applikation von 0,6 mmol Substanz/kg Körpergewicht beobachtet wird. In der Spalte B werden Daten zur akuten Toxizität ($LD_{50}$; Maus p. o.) wiedergegeben.

Tabelle 1

| Verb. Nr. | Änderung der Blutglucosekonzentration (in %) in vivo<br>A | Akute Toxizität Maus p. o. $LD_{50}$ (mg/kg)<br>B |
|---|---|---|
| 1 | − 8 | 475 |
| 2 | − 6 | 410*) |
| 3 | −24 | 1400 |
| 4 | −48 | 520 |
| 5 | −15 | 2400 |
| 6 | −21 | 1600 |
| 7 | −30 | 570 |
| 8 | −22 | 790 |
| 9 | −18 | 330 |

*) Zit. Blickens, D. A., Riggi, S. J.: Toxicol. Appl. Pharmacol. 14 (1969) 393—400.

Zu Tabelle I:

Spalte A = Maximale Änderung der Blutglucosekonzentration (in % bezogen auf die Kontrolltiere) in vivo an nüchternen Ratten, nach Gabe von 0,6 mmol/kg
Spalte B = Akute Toxizität ($LD_{50}$ in mg/kg; Maus p. o.)

Die pharmakologischen Eigenschaften werden nach folgenden Methoden ermittelt:

1. Blutglucosebestimmung nach einmaliger oraler Applikation

Zur Verwendung kommen junge männliche Sprague-Daqley-Ratten (Körpergewicht 150—200 g). Die Haltung der Tiere (6 Tiere pro Dosis) erfolgt in Makrolon-Käfigen mit bis zu 4 Tieren pro Käfig (Raumtemperatur 23° C, rel. Luftfeuchtigkeit 55%, fester Tag/Nacht-Rhythmus (12/12=h) Standarddiät Altromin®). Den Ratten wird 18 Stunden vor der 1. Blutentnahme das Futter entzogen. Wasseraufnahme erfolgt ad libitum. Blutentnahmen erfolgen unmittelbar vor und 2,4 und 6 Stunden nach Substanzgabe durch Punktion aus dem retroorbitalen Plexus. Nach Enteiweißung mit Perchlorsäure erfolgt die Blutglucosebestimmung mittels der enzymatischen HK/G-6-PDH-Methode nach R. Richterich (Klinische Chemie, Theorie und Praxis, 3. Aufl. 1971, S. Karger Verlag, Zürich—Basel, Seite 275). Zum Vergleich wird jeweils eine mit reinem Lösungsmittel behandelte Kontrollgruppe mituntersucht (10 Tiere pro Kontrollgruppe).

2. Bestimmung der Toxizität

Die Toxizitätsuntersuchungen werden an weiblichen NMRI-Mäusen (Körpergewicht 22—26 g) durchgeführt. Die Tiere (5 Tiere pro Dosis) erhalten 18 Stunden vor der Behandlung das Futter (Altromin®) auf 50 g/50 Tiere reduziert und Wasser ad libitum. Verschiedene Dosen der Substanzen werden oral mittels Schlundsonde verabreicht (Volumen 10 ml/kg). Die Beobachtungsdauer beträgt 7 Tage. Die $LD_{50}$, d. h. die Dosis, bei der 50% der Tiere sterben, wird aus der Dosiswirkungskurve graphisch ermittelt.

**Patentansprüche für die Vertragsstaaten: BE, CH, DE, FR, GB, IT, LI, LU, NL, SE**

1. Epoxi-cycloalkylalkancarbonsäuren der allgemeinen Formel I

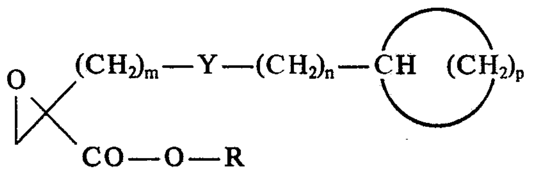

(I)

worin

R ein Wasserstoffatom oder eine Niederalkylgruppe ($C_1$—$C_4$),
Y ein Sauerstoffatom (—O—) oder eine Methylengruppe (—$CH_2$—),
m eine ganze Zahl von 0 bis 6,
n eine ganze Zahl von 0 bis 6 und
p eine ganze Zahl von 2 bis 11

bedeutet (wobei m nicht 0 oder 1 sein kann, wenn Y ein Sauerstoffatom darstellt und wobei die Summe der Zahlen m, n und p eine ganze Zahl von 6 bis 15 ist), sowie die Salze der Carbonsäuren.

2. Epoxi-cycloalkylalkancarbonsäuren der allgemeinen Formel I*

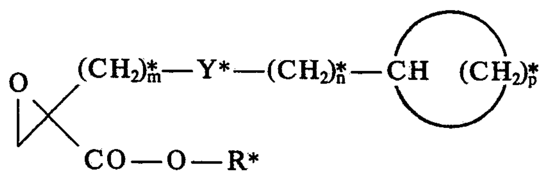

(I*)

worin

R* ein Wasserstoffatom oder eine Niederalkylgruppe ($C_1$—$C_4$),
Y* eine Methylengruppe (—$CH_2$—),
m* eine ganze Zahl von 1 bis 6,
n* die Zahl 1 und
p* eine ganze Zahl von 4 bis 7

bedeutet (wobei die Summe der Zahlen m*, n* und p* eine ganze Zahl von 6 bis 11 ist), sowie die Salze der Carbonsäuren.

3. Verbindungen nach Anspruch 2, gekennzeichnet durch die allgemeine Formel I*, worin R* ein Wasserstoffatom, eine Methyl- oder Ethylgruppe, Y* eine Methylengruppe (—$CH_2$—), m* eine ganze Zahl von 2 bis 4, n* die Zahl 1 und p* die Zahl 5 bedeutet, sowie die Salze der Carbonsäuren.

4. Epoxi-cycloalkylalkancarbonsäuren der allgemeinen Formel I**

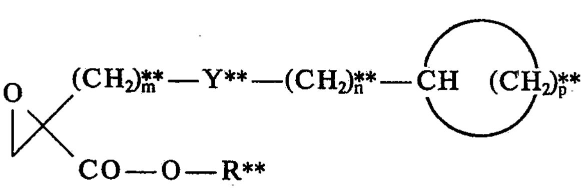

(I**)

worin

R** ein Wasserstoffatom oder eine Niederalkylgruppe ($C_1$—$C_4$),
Y** ein Sauerstoffatom (—O—)
m** eine ganze Zahl von 2 bis 6,
n** eine ganze Zahl von 0 bis 4 und
p** eine ganze Zahl von 4 bis 7

bedeutet (wobei die Summe der Zahlen m**, n** und p** eine ganze Zahl von 6 bis 11 ist), sowie die Salze der Carbonsäuren.

5. Verbindungen nach Anspruch 4, gekennzeichnet durch die allgemeine Formel I**, worin R** ein Wasserstoffatom, eine Methyl- oder Ethylgruppe, Y** ein Sauerstoffatom (—O—), m** eine ganze Zahl von 2 bis 5, n** eine ganze Zahl von 0 bis 3 und p** die Zahl 5 bedeutet, (wobei die Summe aus m** und n** eine Zahl von 3 bis 5 ist), sowie die Salze der Carbonsäuren.

6. 2-(5-Cyclohexylpentyl)-oxiran-2-carbonsäureethylester.

7. 2-[2-(3-Cyclohexylpropyloxy)-ethyl]-oxiran-2-carbonsäureethylester.

8. Arzneimittel enthaltend eine oder mehrere Verbindungen nach einem oder mehreren der Ansprüche 1 bis 7.

9. Verbindungen nach einem oder mehreren der Ansprüche 1 bis 7 zur Anwendung bei der Behandlung und Prophylaxe von Krankheiten, die auf Störungen des Glucose- und Fettstoffwechsels beruhen.

10. Verfahren zur Herstellung von Epoxi-cycloalkylalkancarbonsäuren nach Anspruch 1, dadurch gekennzeichnet, daß man substituierte $\alpha$-Methylencarbonsäuren der allgemeinen Formel II

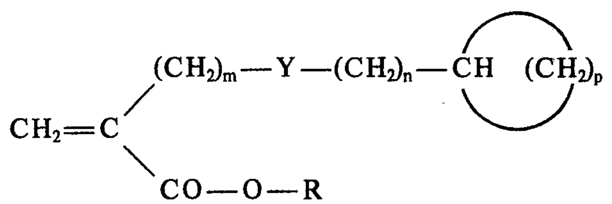

(II)

worin R, Y, m, n und p die in Anspruch 1 angegebene Bedeutung haben, oxidiert und gegebenenfalls anschließend die erhaltenen Niederalkylester verseift und gewünschtenfalls anschließend in die Salze überführt oder die erhaltenen Säuren in die Salze oder Niederalkylester überführt.

**Patentansprüche für den Vertragsstaat: AT**

1. Verfahren zur Herstellung von Epoxi-cycloalkylalkancarbonsäuren der allgemeinen Formel I

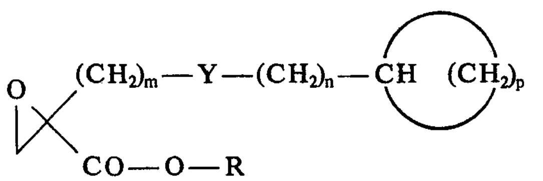

(I)

worin

R ein Wasserstoffatom oder eine Niederalkylgruppe ($C_1-C_4$),
Y ein Sauerstoffatom (—O—) oder eine Methylengruppe ($-CH_2-$),
m eine ganze Zahl von 0 bis 6,
n eine ganze Zahl von 0 bis 6 und
p eine ganze Zahl von 2 bis 11

bedeutet (wobei m nicht 0 oder 1 sein kann, wenn Y ein Sauerstoffatom ist und wobei die Summe der Zahlen m, n und p eine ganze Zahl von 6 bis 15 ist), sowie den Salzen der Carbonsäuren, dadurch gekennzeichnet, daß man substituierte $\alpha$-Methylencarbonsäuren der allgemeinen Formel II

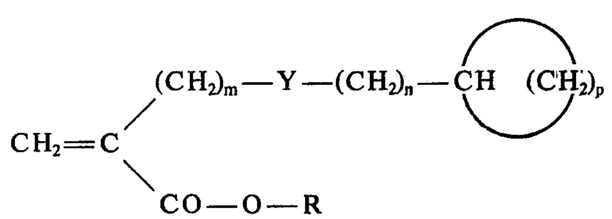

(II)

worin R, Y, m, n und p die vorstehend angegebene Bedeutung haben, oxidiert und gegebenenfalls anschließend die erhaltenen Niederalkylester verseift und gewünschtenfalls anschließend in die Salze überführt oder die erhaltenen Säuren in die Salze oder Niederalkylester überführt.

2. Verfahren zur Herstellung von Epoxi-cycloalkylalkancarbonsäuren der allgemeinen Formel I*

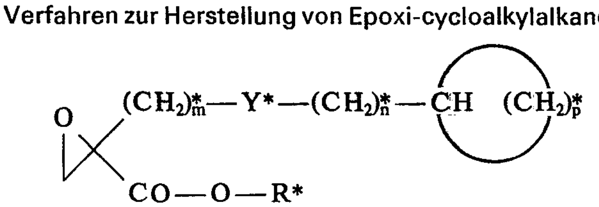

(I*)

worin

R* ein Wasserstoffatom oder eine Niederalkylgruppe ($C_1-C_4$),
Y* eine Methylengruppe ($-CH_2-$),
m* eine ganze Zahl von 1 bis 6,
n* die Zahl 1 und
p* eine ganze Zahl von 4 bis 7

bedeutet (wobei die Summe der Zahlen m*, n* und p* eine ganze Zahl von 6 bis 11 ist), sowie den Salzen der Carbonsäuren, dadurch gekennzeichnet, daß man substituierte $\alpha$-Methylencarbonsäuren der allgemeinen Formel II*

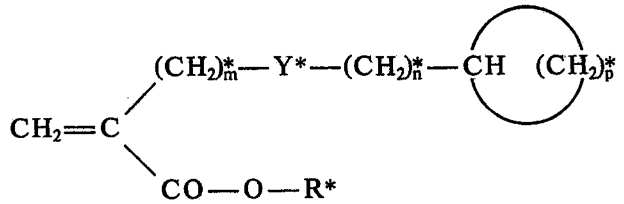

(II*)

worin R*, Y*, m*, n* und p* die vorstehend angegebenen Bedeutungen haben, oxidiert und gegebenenfalls anschließend die erhaltenen Niederalkylester verseift und gewünschtenfalls anschließend in die Salze überführt oder die erhaltenen Säuren in die Salze oder Niederalkylester überführt.

3. Verfahren nach Anspruch 2, wobei R* ein Wasserstoffatom, eine Methyl- oder Ethylgruppe, Y* eine Methylengruppe ($-CH_2-$), m* eine ganze Zahl von 2 bis 4, n* die Zahl 1 und p* die Zahl 5 bedeutet.

4. Verfahren zur Herstellung von Epoxi-cycloalkylalkancarbonsäuren der allgemeinen Formel I**

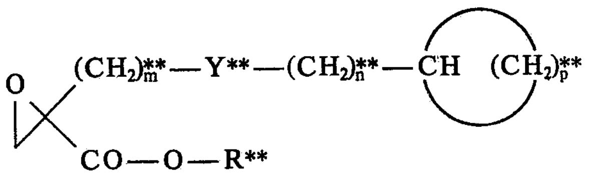

(I**)

worin

R** ein Wasserstoffatom oder eine Niederalkylgruppe ($C_1-C_4$),
Y** ein Sauerstoffatom ($-O-$)
m** eine ganze Zahl von 2 bis 6,
n** eine ganze Zahl von 0 bis 4 und
p** eine ganze Zahl von 4 bis 7

bedeutet (wobei die Summe der Zahlen m**, n** und p** eine ganze Zahl von 6 bis 11 ist), sowie den Salzen der Carbonsäuren, dadurch gekennzeichnet, daß man substituierte $\alpha$-Methylencarbonsäuren der allgemeinen Formel II**

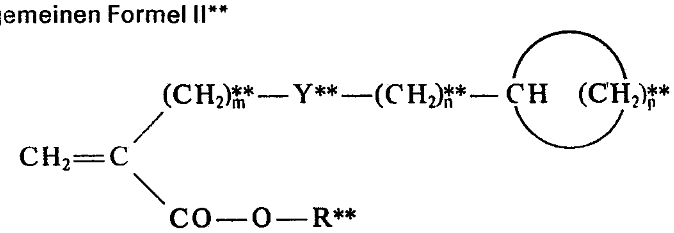

(II**)

worin R**, Y**, m**, n** und p** die vorstehend angegebenen Bedeutungen haben, oxidiert und gegebenenfalls anschließend die erhaltenen Niederalkylester verseift und gewünschtenfalls anschließend in die Salze überführt oder die erhaltenen Säuren in die Salze oder Niederalkylester überführt.

5. Verfahren nach Anspruch 4, wobei R** ein Wasserstoffatom, eine Methyl- oder Ethylgruppe, Y** ein Sauerstoffatom, m** eine ganze Zahl von 2 bis 5, n** eine ganze Zahl von 0 bis 3 und p** die Zahl 5 bedeutet und wobei die Summe aus m** und n** eine Zahl von 3 bis 5 ist.

6. Verfahren nach einem oder mehreren der Ansprüche 1 bis 5, dadurch gekennzeichnet, daß man 2-(5-Cyclohexylpentyl)-oxiran-2-carbonsäureethylester herstellt.

7. Verfahren nach einem oder mehreren der Ansprüche 1 bis 5, dadurch gekennzeichnet, daß man 2-[2-(3-Cyclohexylpropyloxy)-ethyl]-oxiran-2-carbonsäureethylester herstellt.

**Claims for the contracting states: BE, CH, DE, FR, GB, IT, LI, LU, NL, SE:**

1. Epoxycycloalkylalkanecarboxylic acids of the general formula I

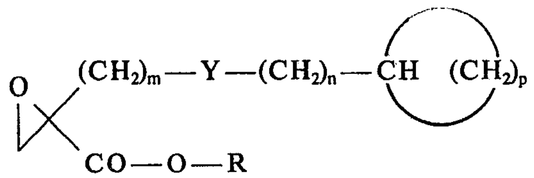

(I)

wherein

R denotes a hydrogen atom or a lower alkyl group ($C_1-C_4$),
Y denotes an oxygen atom ($-O-$) or a methylene group ($-CH_2-$),
m denotes an integer from 0 to 6,
n denotes an integer from 0 to 6 and
p denotes an integer from 2 to 11

(and m cannot be 0 or 1 if Y represents an oxygen atom, and the sum of the numbers m, n and p is an integer from 6 to 15), and also the salts of the carboxylic acids.

2. Epoxycycloalkylalkanecarboxylic acids of the general formula I*

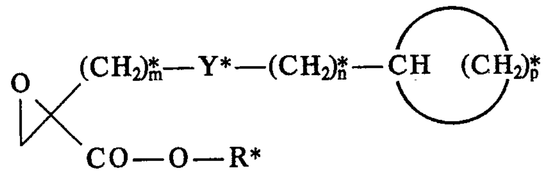

(I*)

wherein

R* denotes a hydrogen atom or a lower alkyl group ($C_1-C_4$),
Y* denotes a methylene group ($-CH_2-$),
m* denotes an integer from 1 to 6,
n* denotes the number 1 and
p* denotes an integer from 4 to 7

(and the sum of the numbers m*, n* and p* is an integer from 6 to 11), and also the salts of the carboxylic acids.

3. Compounds according to Claim 2, characterized by the general formula I*, wherein R* denotes a hydrogen atom, a methyl- or ethyl group, Y* denotes a methylene group ($-CH_2-$), m* denotes an integer from 2 to 4, n* denotes 1 and p* denotes 5, and also the salts of the carboxylic acids.

4. Epoxycycloalkylalkanecarboxylic acids of the general formula I**

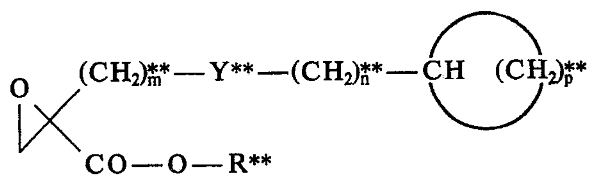

(I**)

wherein

R** denotes a hydrogen atom or a lower alkyl group ($C_1-C_4$),
Y** denotes an oxygen atom ($-O-$),
m** denotes an integer from 2 to 6,
n** denotes an integer from 0 to 4 and
p** denotes an integer from 4 to 7

(and the sum of the numbers m**, n** and p** is an integer from 6 to 11), and also the salts of the carboxylic acids.

5. Compounds according to Claim 4, characterized by the general formula I**, wherein R** denotes a hydrogen atom, a methyl- or ethyl group, Y** denotes oxygen ($-O-$), m** denotes an intege from 2 to 5, n** denotes an integer from 0 to 3 and p** denotes 5 (and the sum of the numbers m** and n** is an integer from 3 to 5), and also the salts of the carboxylic acids.

6. 2-(5-Cyclohexylpentyl)-oxirane-2-carboxylic acid ethyl ester.

7. 2-[2-(3-Cyclohexylpropyloxy)-ethyl]-oxirane-2-carboxylic acid ethyl ester.

8. Medicaments containing one or more compounds according to one or more of the Claims 1 to 7.

9. Use of compounds according to one or more of the Claims 1 to 7 in the treatment and prophylaxis of diseases caused by disturbances of the metabolism of glucose and fats.

10. Process for the preparation of epoxycycloalkylalkanecarboxylic acids according to Claim 1, characterized in that a substituted $\alpha$-methylenecarboxylic acid of the general formula II

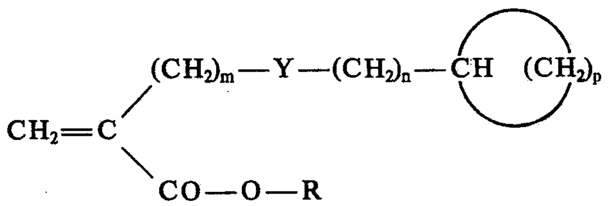

(II)

wherein R, Y, m, n and p have the meanings indicated in Claim 1 is oxidised and, if appropriate, the resulting lower alkyl esters are then saponified and, if desired, subsequently converted into the salts or the resulting acids are converted into the salts or lower alkyl esters.

**Claims for the contracting state: AT**

1. Process for the preparation of epoxycycloalkylalkanecarboxylic acids of the general formula I

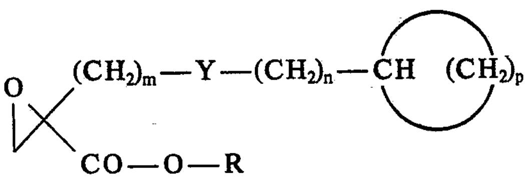

(I)

wherein

R denotes a hydrogen atom or a lower alkyl group ($C_1-C_4$),
Y denotes an oxygen atom ($-O-$) or a methylene group ($-CH_2-$),
m denotes an integer from 0 to 6,
n denotes an integer from 0 to 6 and
p denotes an integer from 2 to 11

(and m cannot be 0 or 1 if Y represents an oxygen atom, and the sum of the numbers m, n and p is an integer from 6 to 15), and also the salts of the carboxylic acids, characterized in that a substituted $\alpha$-methylenecarboxylic acid of the general formula II

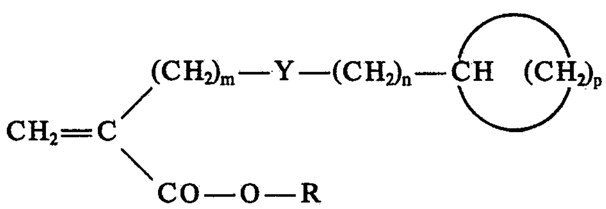

(II)

wherein R, Y, m, n and p have the meanings indicated in Claim 1 is oxidised and, if appropriate, the resulting lower alkyl esters are then saponified and, if desired, subsequently converted into the salts or the resulting acids are converted into the salts or lower alkyl esters.

2. Process for the preparation of epoxycycloalkylalkanecarboxylic acids of the general formula I*

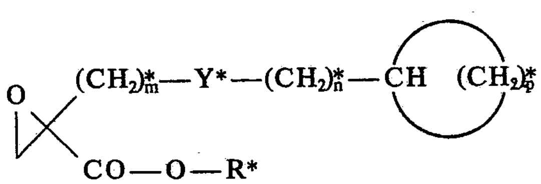

(I*)

wherein

R* denotes a hydrogen atom or a lower alkyl group ($C_1-C_4$),
Y* denotes a methylene group ($-CH_2-$),
m* denotes an integer from 1 to 6,
n* denotes the number 1 and
p* denotes an integer from 4 to 7

(and the sum of the numbers m*, n* and p* is an integer from 6 to 11), and also the salts of the carboxylic acids, characterized in that a substituted $\alpha$-methylenecarboxylic acid of the general formula II*

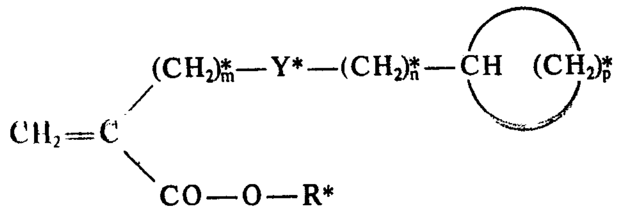

(II*)

wherein R*, Y*, m*, n* and p* have the meanings indicated above, is oxidised and, if appropriate, the resulting lower alkyl esters are then saponified and, if desired, are then converted into the salts, or the resulting acids are converted into the salts or lower alkyl esters.

3. Process according to Claim 2, wherein R* denotes a hydrogen atom, a methyl or ethyl group, Y* denotes a methylene group ($-CH_2-$), m* denotes an integer from 2 to 4, n* denotes 1 and p* denotes 5.

4. Process for the preparation of epoxycycloalkylalkanecarboxylic acids of the general formula I**

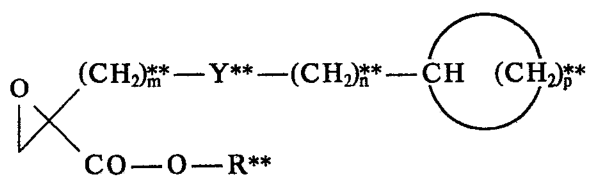

(I**)

wherein

R** denotes a hydrogen atom or a lower alkyl group ($C_1-C_4$),
Y** denotes an oxygen atom ($-O-$),
m** denotes an integer from 2 to 6,
n** denotes an integer from 0 to 4 and
p** denotes an integer from 4 to 7

(and the sum of the numbers m**, n** and p** is an integer from 6 to 11), and also the salts of the carboxylic acids, characterised in that a substituted $\alpha$-methylenecarboxylic acid of the general formula II**

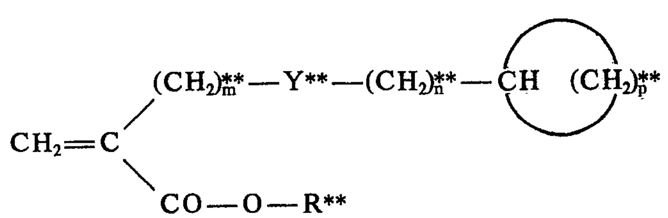

(II**)

wherein R**, Y**, m**, n** and p** have the meanings indicated above, is oxidised and, if appropriate, the resulting lower alkyl esters are then saponified and, if desired, then converted into the salts, or the resulting acids are converted into the salts or lower alkyl esters.

5. Process according to Claim 4, wherein R** denotes a hydrogen atom, a methyl or ethyl group, Y** denotes oxygen, m** denotes an integer from 2 to 5, n** denotes an integer from 0 to 3 and p** denotes 5 and the sum of the numbers m** and n** is an integer from 3 to 5.

6. Process according to one or more of the claims 1 to 5, characterized in, that 2-(5-Cyclohexylpentyl)-oxirane-2-carboxylic acid ethyl ester is prepared.

7. Process according to one or more of the claims 1 to 5, chartacterized in, that 2-[2-(3-Cyclohexylpropyloxy)-ethyl]-oxirane-2-carboxylic acid ethyl ester is prepared.

**Revendications pour les Etats contractants: BE, CH, DE, FR, GB, IT, LI, LU, NL, SE**

1. Acides époxy-cycloalkylalcane-carboxyliques de formule générale I ci-après:

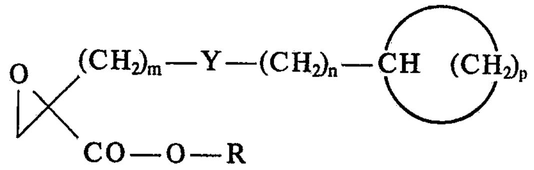

(I)

dans laquelle

R représente un atome d'hydrogène ou un groupe alkyle inférieur (en $C_1$ à $C_4$),
Y représente un atome d'oxygène ($-O-$) ou un groupe méthylène ($-CH_2-$),
m représente un nombre entier compris entre 0 et 6,
n représente un nombre entier compris entre 0 et 6 et
p représente un nombre entier compris entre 2 et 11,

(m ne pouvant pas être égal à 0 ou à 1 quand Y représente un atome d'oxygène et la somme des nombres m, n et p étant un nombre entier compris entre 6 et 15), ainsi que les sels des acides carboxyliques.

2. Acides époxy-cycloalkylalcane-carboxyliques de formule générale I* ci-après:

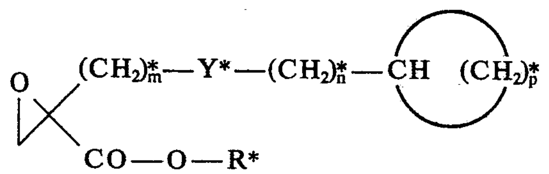

(I*)

dans laquelle

R* représente un atome d'hydrogène ou un groupe alkyle inférieur (en $C_1$ à $C_4$),
Y* représente un groupe méthylène ($-CH_2-$),
m* représente un nombre entier compris entre 1 et 6,
n* est égal à 1 et
p* représente un nombre entier compris entre 4 et 7

(la somme des nombres m*, n* et p* étant un nombre entier compris entre 6 et 11), ainsi que les sels des acides carboxyliques.

3. Composés selon la revendication 2, caractérisés par la formule générale I*, dans laquelle R* représente un atome d'hydrogène, un groupe méthyle ou éthyle, Y* représente un groupe méthylène ($-CH_2-$), m* représente un nombre entier compris entre 2 et 4, n* est égal à 1 et p* est égal à 5, ainsi que les sels des acides carboxyliques.

4. Acides époxy-cycloalkylalcane-carboxyliques de formule générale I** ci-après:

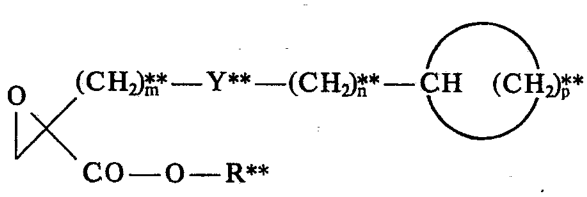

(I**)

dans laquelle

R** représente un atome d'hydrogène ou un groupe alkyle inférieur (en $C_1$ à $C_4$),
Y** représente un atome d'oxygène ($-O-$),
m** représente un nombre entier compris entre 2 et 6,
n** représente un nombre entier compris entre 0 et 4 et
p** représente un nombre entier compris entre 4 et 7,

(la somme des nombres m**, n** et p** étant un nombre entier compris entre 6 et 11), ainsi que les sels des acides carboxyliques.

5. Composés selon la revendication 4, caractérisés par la formule générale I**, dans laquelle R** représente un atome d'hydrogène, un groupe méthyle ou éthyle, Y** représente un atome d'oxygène ($-O-$), m** représente un nombre entier compris entre 2 et 5, n** représente un nombre entier compris entre 0 et 3 et p** est égal à 5, (la somme de m** et n** étant un nombre entier compris entre 3

et 5), ainsi que les sels des acides carboxyliques.

6. Ethylester de l'acide 2-(5-cyclohexylpentyl)-oxyrane-2-carboxylique.

7. Ethylester de l'acide 2-[2-(3-cyclohexylpropyloxy) éthyl]-oxyrane 2 carboxylique

8. Médicament contenant un ou plusieurs composés selon une ou plusieurs des revendications 1 à 7.

9. Composés selon une ou plusieurs des revendications 1 à 7, pour l'utilisation dans le traitement ou la prévention de maladies qui reposent sur des troubles du métabolisme des sucres et des graisses.

10. Procédé de préparation d'acides époxy-cycloalkylalcane-carboxyliques selon la revendication 1, caractérisé en ce que l'on oxyde des acides $\alpha$-méthylènecarboxyliques substitués de formule générale II:

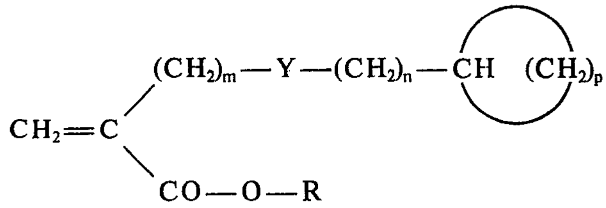

(II)

dans laquelle R, Y, m, n et p ont la signification donnée dans la revendication 1, après quoi l'on saponifie le cas échéant les alkylesters inférieurs obtenus, et on les transforme ensuite le cas échéant en les sels ou bien on transforme les acides obtenus en les sels ou en les alkylesters inférieurs.

**Revendications pour l'Etat contractant: AT**

1. Procédé de préparation d'acides époxy-cycloalkylalcane-carboxyliques de formule générale I ci-après:

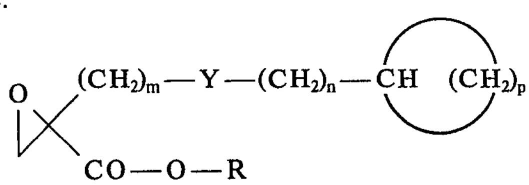

(I)

dans laquelle

R représente un atome d'hydrogène ou un groupe alkyle inférieur (en $C_1$ à $C_4$),
Y représente un atome d'oxygène ($-O-$) ou un groupe méthylène ($-CH_2-$),
m représente un nombre entier compris entre 0 et 6,
n représente un nombre entier compris entre 0 et 6 et
p représente un nombre entier compris entre 2 et 11,

(m ne pouvant pas être égal à 0 ou à 1 quand Y représente un atome d'oxygène et la somme des nombres m, n et p étant un nombre entier compris entre 6 et 15), ainsi que les sels des acides carboxyliques, caractérisé en ce que l'on oxyde des acides $\alpha$-méthylènecarboxyliques substitués de formule générale II ci-après:

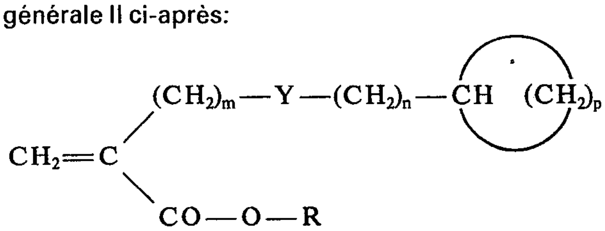

(II)

dans laquelle R, Y, m, n et p ont la même signification que ci-dessus, puis l'on saponifie ensuite le cas échéant les alkylesters inférieurs obtenus et on les transforme ensuite le cas échéant en leurs sels ou bien l'on transforme les acides obtenus en leurs sels ou en leurs alkylesters inférieurs.

2. Procédé de préparation d'acides époxy-cycloalkylalcane-carboxyliques de formule générale I* ci-après:

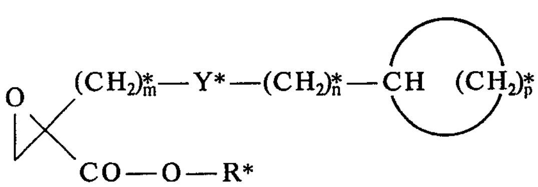

(I*)

dans laquelle

R* représente un atome d'hydrogène ou un groupe alkyle inférieur (en $C_1$ à $C_4$),
Y* représente un groupe méthylène ($-CH_2-$),
m* représente un nombre entier compris entre 1 et 6,
n* est égal à 1 et
p* représente un nombre entier compris entre 4 et 7

(la somme des nombres m*, n* et p* étant un nombre entier compris entre 6 et 11), ainsi que les sels des acides carboxyliques, caractérisé en ce que l'on oxyde des acides $\alpha$-méthylènecarboxyliques substitués de formule générale II* ci-après:

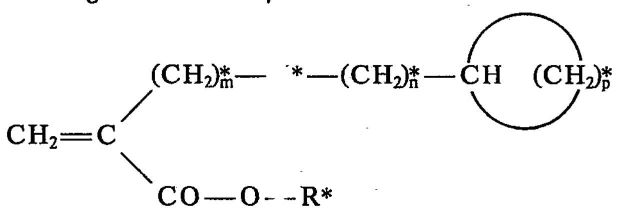

(II*)

dans laquelle R*, Y*, m*, n* et p* ont la même signification que ci dessus, et l'on saponifie le cas échéant ensuite les alkylesters inférieurs obtenus et on les transforme ensuite, le cas échéant en leurs sels, ou bien on transforme les acides obtenus en leurs sels ou en leurs alkylesters inférieurs.

3. Procédé selon la revendication 2, caractérisé en ce que R* représente un atome d'hydrogène, un groupe méthyle ou éthyle, Y* représente un groupe méthylène ($-CH_2-$), m* représente un nombre entier compris entre 2 et 4, n* est égal à 1 et p* est égal à 5.

4. Procédé de préparation d'acides époxy-cycloalkylalcane-carboxyliques de formule générale I** ci-après:

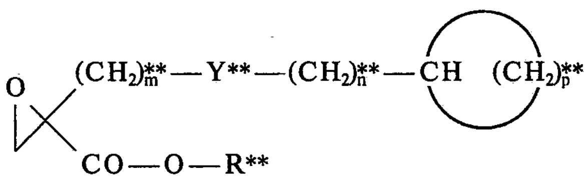

(I**)

dans laquelle

R** représente un atome d'hydrogène ou un groupe alkyle inférieur (en $C_1$ à $C_4$),
Y** représente un atome d'oxygène ($-O-$),
m** représente un nombre entier compris entre 2 et 6,
n** représente un nombre entier compris entre 0 et 4 et
p** représente un nombre entier compris entre 4 et 7,

(la somme des nombres m**, n** et p** étant un nombre entier compris entre 6 et 11), ainsi que les sels des acides carboxyliques, caractérisé en ce que l'on oxyde des acides $\alpha$-méthylènecarboxyliques substitués de formule générale II** ci-après:

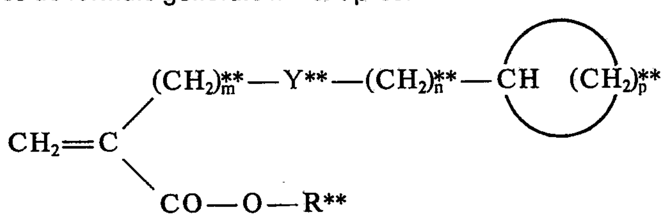

(II**)

dans laquelle R**, Y**, m**, n** et p** ont les mêmes significations que ci-dessus et on saponifie ensuite le cas échéant les alkylesters inférieurs obtenus et on les transforme ensuite, le cas échéant, en leurs sels, ou l'on transforme les acides obtenus en leurs sels ou en leurs alkylesters inférieurs.

5. Procédé selon la revendication 4, caractérisé en ce que R** représente un atome d'hydrogène, un groupe méthyle ou éthyle, Y** représente un atome d'oxygène, m** représente un nombre entier compris entre 2 et 5, n** représente un nombre entier compris entre 0 et 3 et p** est égal à 5 et la somme de m** et n** est un nombre entier compris entre 3 et 5.

6. Procédé selon une ou plusieurs des revendications 1 à 5, caractérisé en ce que l'on prépare l'éthylester de l'acide 2-(5-cyclohexylpentyl)-oxyrane-2- carboxylique.

7. Procédé selon une ou plusieurs des revendications 1 à 5, caractérisé en ce que l'on prépare l'éthylester de l'acide 2-[2-(3-cyclohexylpropyloxy)-éthyl]-oxyrane-2-carboxylique.